(19) Europäisches Patentamt
European Patent Office
Office européen des brevets

(11) EP 4 116 292 A1

(12) # EUROPEAN PATENT APPLICATION
published in accordance with Art. 153(4) EPC

(43) Date of publication:
**11.01.2023 Bulletin 2023/02**

(21) Application number: **21765257.7**

(22) Date of filing: **05.03.2021**

(51) International Patent Classification (IPC):
***C07D 233/56** (2006.01)* ***A01N 43/50** (2006.01)*
***A01N 43/653** (2006.01)* ***A01N 47/02** (2006.01)*
***A01P 1/00** (2006.01)* ***A01P 3/00** (2006.01)*
***C07D 249/08** (2006.01)* ***C07D 401/12** (2006.01)*
***C07D 403/12** (2006.01)*

(52) Cooperative Patent Classification (CPC):
**A01N 43/50; A01N 43/653; A01N 47/02; A01P 1/00; A01P 3/00; C07D 233/56; C07D 249/08; C07D 401/12; C07D 403/12**

(86) International application number:
**PCT/JP2021/008690**

(87) International publication number:
**WO 2021/177442 (10.09.2021 Gazette 2021/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.03.2020 JP 2020039353**

(71) Applicant: **Kureha Corporation**
**Chuo-ku**
**Tokyo 103-8552 (JP)**

(72) Inventors:
- **MASANO, Taiga**
  **Tokyo 103-8552 (JP)**
- **MUKADE, Tsutomu**
  **Tokyo 103-8552 (JP)**
- **KOUGE, Tomoyuki**
  **Tokyo 103-8552 (JP)**
- **MIYAKE, Taiji**
  **Tokyo 103-8552 (JP)**
- **HIRATA, Junya**
  **Tokyo 103-8552 (JP)**

(74) Representative: **Zimmermann & Partner Patentanwälte mbB**
**Postfach 330 920**
**80069 München (DE)**

(54) **AZOLE DERIVATIVE, METHOD FOR PRODUCING AZOLE DERIVATIVE, AGENT FOR AGRICULTURAL AND HORTICULTURAL USE, AND INDUSTRIAL MATERIAL PROTECTION AGENT**

(57) Provided is a plant disease controlling agent that has low toxicity to humans and animals, that is excellent in handling safety, and that has an excellent controlling effect against a wide range of plant diseases and a high antimicrobial action against plant disease fungi. The present invention is a compound represented by general formula (I) below, or N-oxide or a pesticidally acceptable salt thereof:

EP 4 116 292 A1

[Chem. 1]

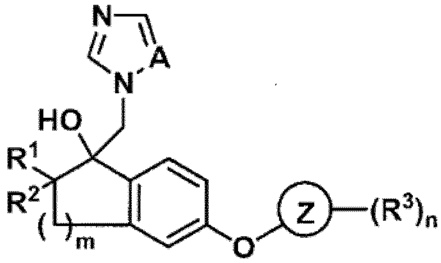

(I)

## Description

### [TECHNICAL FIELD]

The present invention relates to a new azole derivative and a method for producing an azole derivative. Furthermore, the present invention relates to an agricultural or horticultural chemical and an industrial material protectant containing the azole derivative as an active ingredient.

### [BACKGROUND ART]

To-date, agricultural and horticultural chemicals having low toxicity toward humans and animals and excellent safety in handling, and exhibiting a high controlling effect against a wide variety of plant diseases have been demanded. An azole fungicide is known as an agricultural or horticultural chemical exhibiting a high controlling effect.

[Citation List]

[Patent Document]

Patent Document 1: WO 2013/007767
Patent Document 2: WO 2019/093522

### [SUMMARY OF INVENTION]

[Technical Problem]

There is a demand for a plant disease controlling agent that has low toxicity to humans and animals, that is excellent in handling safety, and that has an excellent controlling effect against a wide range of plant diseases and a high antimicrobial action against plant disease fungi.
The present invention is completed in light of the problems described above. An objective of the present invention is to provide a compound that meets the above requirements.

[Solution to Problem]

As a result of diligent research to solve the above problems, the present inventors have found that an azole derivative represented by general formula (I) below have excellent activity, and thus have completed the present invention.
The azole derivative according to an embodiment of the present invention is a compound represented by general formula (I) below, or N-oxide or a pesticidally acceptable salt thereof:

[Chem. 1]

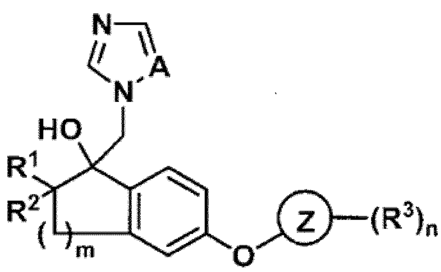

(I)

[In Formula (I), A is N or CH;

$R^1$ and $R^2$ are each independently hydrogen, a $C_1$-$C_6$-alkyl group, a $C_3$-$C_8$-cycloalkyl group, or a $C_3$-$C_8$-cycloalkyl-$C_1$-$C_4$-alkyl group;
$R^1$ and $R^2$ may be bonded to each other to form a ring;

Z is a phenyl group or a 5- or 6-membered aromatic heterocyclic ring containing 1, 2, 3, or 4 heteroatoms selected from O, N, and S;
$R^3$ is halogen, a hydroxy group, an amino group, a nitrile group, a nitro group, a pentafluorosulfanyl group, a $C_1$-$C_4$-alkyl group, a $C_1$-$C_4$-haloalkyl group, a $C_1$-$C_4$-alkoxy group, or a $C_1$-$C_4$-haloalkoxy group;
$R^3$ is bonded to any substitution position of Z in a number of n;
n is 0, 1, 2, 3, 4, or 5; and
m is 1 or 2].

[Advantageous Effects of Invention]

An azole derivative according to an embodiment of the present invention exhibits excellent fungicidal activity against many types of fungi that cause diseases in plants. Therefore, the chemical containing the azole derivative according to an embodiment of the present invention as an active ingredient exhibits a high controlling effect against a wide variety of plant diseases.

[Description of Embodiments]

A preferred embodiment for carrying out the present invention will now be explained. Moreover, the embodiment explained below illustrates a single representative example of the present invention, and it should not be interpreted that the scope of the present invention is narrowed by this embodiment.

[1. Azole derivative]

The azole derivative according to an embodiment of the present invention is an azole derivative represented by the following general formula (I) below (hereinafter, referred to as "azole derivative (I)").

[Chem. 2]

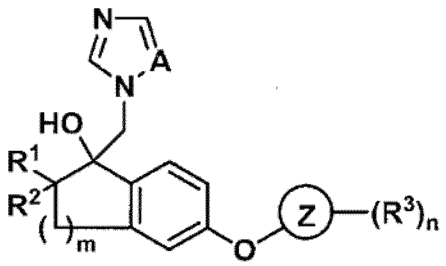

(I)

In general formula (I), A is N or CH, and preferably N.

$R^1$ and $R^2$ are each independently hydrogen, a $C_1$-$C_6$-alkyl group, a $C_3$-$C_8$-cycloalkyl group, or a $C_3$-$C_8$-cycloalkyl-$C_1$-$C_4$-alkyl group.

The $C_1$-$C_6$-alkyl group is a linear or branched alkyl group having 1 to 6 carbon atoms, and examples thereof include a methyl group, an ethyl group, a 1-methylethyl group, a 1,1-dimethylethyl group, a propyl group, a 1-methylpropyl group, a 2-methylpropyl group, a 1,1-dimethylpropyl group, a 2,2-dimethylpropyl group, a 1-ethyl propyl group, a butyl group, a 1-methylbutyl group, a 2-methylbutyl group, a 3-methylbutyl group, a 3,3-dimethylbutyl group, a 2,2-dimethylbutyl group, a 1,1-dimethylbutyl group, a 1-ethylbutyl group, a 2-ethyl butyl group, a pentyl group, a 1-methylpentyl group, a 2-methylpentyl group, a 3-methylpentyl group, and a 4-methylpentyl group.

The $C_3$-$C_8$-cycloalkyl group is a cyclic alkyl having 3 to 8 carbon atoms, and examples thereof include a cyclopropyl group, a cyclobutyl group, a cyclopentyl group, a cyclohexyl group, a cycloheptyl group, and a cyclooctyl group.

The $C_3$-$C_8$-cycloalkyl-$C_1$-$C_4$-alkyl group indicates that a cyclic cycloalkyl group having 3 to 8 carbon atoms is bonded to a linear or branched alkyl group having 1 to 4 carbons. Examples thereof include a cyclopropylmethyl group, a cyclobutylmethyl group, a cyclopentylmethyl group, a cyclohexylmethyl group, a 2-cyclopropylethyl group, a 1-cyclopropylethyl group, a 2-cyclohexylethyl group, a 3-cyclopropylpropyl group, a 2-cyclopropylpropyl group, and 4-cyclopropylbutyl group.

$R^1$ and $R^2$ may be bonded to each other to form a ring together with a carbon atom to which $R^1$ and $R^2$ are bonded.

Z is a phenyl group or a 5- or 6-membered aromatic heterocyclic ring containing 1, 2, 3, or 4 heteroatoms.

Here, the heteroatom is an atom selected from O, N, and S. When the aromatic heterocyclic ring contains a plurality of heteroatoms, the plurality of heteroatoms may be the same or different from each other. Z is preferably a phenyl group or a 5- or 6-membered aromatic heterocyclic ring containing 1 to 3 heteroatoms selected from N and S, and is more preferably a phenyl group.

Examples of the 5 or 6 membered aromatic heterocyclic groups include a furyl group, a pyrazolyl group, a thienyl group, a pyridyl group, a pyrimidinyl group, a pyridazinyl group, a pyrazinyl group, a pyrrolyl group, an imidazolyl group, a pyrazolyl group, a thiazolyl group, an isothiazolyl group, an oxazolyl group, an isoxazolyl group, an oxadiazolyl group, a thiadiazolyl group, a triazolyl group, a tetrazolyl group, and a triazinyl group.

At optional positions, n of $R^3$s are bonded to Z. Here, n is 0, 1, 2, 3, 4, or 5. $R^3$ is a halogen, a hydroxy group, an amino group, a nitrile group, a nitro group, a pentafluorosulfanyl group, a $C_1$-$C_4$-alkyl group, a $C_1$-$C_4$-haloalkyl group, a $C_1$-$C_4$-alkoxy group, or a $C_1$-$C_4$-haloalkoxy group. When n is 2 or greater, $R^3$s may be the same or different from each other.

The $C_1$-$C_4$-alkyl group is a linear or branched alkyl group having 1 to 4 carbon atoms, and examples thereof include groups in which the number of carbon atoms in the $C_1$-$C_6$-alkyl group is 4 or less.

The $C_1$-$C_4$-haloalkyl group is a group in which one or two or more halogen atoms have been substituted with a position at which the $C_1$-$C_4$-alkyl group can be substituted. When the halogen groups to be substituted are two or more, the halogen groups may be the same or different from each other. Examples of the halogen group include a chlorine group, a bromine group, an iodine group, and a fluorine group. Examples of the $C_1$-$C_4$-haloalkyl group include a chloromethyl group, a 2-chloroethyl group, a 2,3-dichloropropyl group, a bromomethyl group, a chlorodifluoromethyl group, a trifluoromethyl group, and a 3,3,3-trifluoropropyl group.

The $C_1$-$C_4$-alkoxy group is a linear or branched alkoxy group having 1 to 4 carbon atoms, and examples thereof include a methoxy group, an ethoxy group, an n-propoxy group, an isopropoxy group, an n-butoxy group, a sec-butoxy group, and a tert-butoxy group.

The $C_1$-$C_4$-haloalkoxy group is a group in which one or two or more halogen groups have been substituted with a position at which the $C_1$-$C_4$-alkoxy group can be substituted. When the halogen groups to be substituted are two or more, the halogen groups may be the same or different from each other.

The bonding position of $R^3$ is not limited, and is preferably at a 2-, 3-, or 4-position relative to an ether bond of general formula (I), and more preferably is in the 4-position.

m is 1 or 2. That is, when m = 1, the azole derivative (I) has an indan backbone, and when m = 2, it has a tetralin backbone. m is preferably 1.

A preferable aspect of the azole derivative (I) includes an azole derivative (I) in which $R^1$ and $R^2$ are each independently hydrogen, a $C_1$-$C_6$-alkyl group, a $C_3$-$C_8$-cycloalkyl group, and a $C_3$-$C_8$-cycloalkyl-$C_1$-$C_4$-alkyl group, or an azole derivative (I) in which $R^1$ and $R^2$ are bonded to form a cycloalkyl group. An even more preferable aspect of the azole derivative (I) includes an azole derivative (I) in which m is 1. An even more preferable aspect of the azole derivative (I) further includes an azole derivative (I) in which Z is a phenyl group.

An even more preferable aspect of the azole derivative (I) includes an azole derivative (I) in which $R^1$ and $R^2$ are each independently hydrogen, a $C_1$-$C_6$-alkyl group, or an azole derivative (I) in which $R^1$ and $R^2$ are bonded to form a cycloalkyl group. An even more preferable aspect of the azole derivative (I) includes an azole derivative (I) in which $R^1$ and $R^2$ are each independently hydrogen, a $C_1$-$C_6$-alkyl group, or an azole derivative (I) in which $R^1$ and $R^2$ are bonded to form a cycloalkyl group, and m is 1. An even more preferable aspect of the azole derivative (I) includes an azole derivative (I) in which $R^1$ and $R^2$ are each independently hydrogen, a $C_1$-$C_6$-alkyl group, or an azole derivative (I) in which $R^1$ and $R^2$ are bonded to form a cycloalkyl group, m is 1, and $R^3$ is a halogen, $C_1$-$C_4$-haloalkyl group or $C_1$-$C_4$-haloalkoxy group.

Table 1-1 and Table 1-2 below list azole derivatives exemplified as examples of particularly preferable azole derivatives (I). A, $R^1$, $R^2$ and m in Table 1-1 and Table 1-2 below respectively correspond to A, $R^1$ and $R^2$ and m of the above formula (I), and Z-$(R^3)_n$ in Table 1-1 and Table 1-2 above respectively corresponds to a structural part represented by Z and $(R^3)_n$ in the above formula (I).

[Table 1-1]

| Compound No. | A | $R^1$ | $R^2$ | m | Z-$(R^3)_n$ |
|---|---|---|---|---|---|
| I-1 | CH | $CH_3$ | $CH_3$ | 1 | 4-chlorophenyl |
| I-2 | N | H | H | 2 | 4-chlorophenyl |
| I-3 | N | $CH_3$ | $CH_3$ | 2 | 4-chlorophenyl |
| I-4 | N | $CH_3$ | $CH_3$ | 1 | phenyl |
| I-5 | N | $CH_3$ | $CH_3$ | 1 | 2-chlorophenyl |

(continued)

| Compound No. | A | $R^1$ | $R^2$ | m | Z-$(R^3)_n$ |
|---|---|---|---|---|---|
| I-6 | N | $CH_3$ | $CH_3$ | 1 | 3-chlorophenyl |
| I-7 | N | $CH_3$ | $CH_3$ | 1 | 4-chlorophenyl |
| I-8 | N | $CH_3$ | $CH_3$ | 1 | 2,4-dichlorophenyl |
| I-9 | N | $CH_3$ | $CH_3$ | 1 | 3,4-dichlorophenyl |
| I-10 | N | $CH_3$ | $CH_3$ | 1 | 4-bromophenyl |
| I-11 | N | $CH_3$ | $CH_3$ | 1 | 4-fluorophenyl |
| I-12 | N | $CH_3$ | $CH_3$ | 1 | 3,4-difluorophenyl |
| I-13 | N | $CH_3$ | $CH_3$ | 1 | 4-trifluoromethoxyphenyl |
| I-14 | N | $CH_3$ | $CH_3$ | 1 | 4-trifluoromethylphenyl |
| I-15 | N | $CH_3$ | $CH_3$ | 1 | 6-chloropyridin-3-yl |
| I-16 | N | $CH_3$ | $CH_3$ | 1 | 5-chloropyridin-2-yl |
| I-17 | N | cyclopropyl | | 1 | 4-chlorophenyl |
| I-18 | N | $CH_3$ | $CH_3$ | 1 | 5-trifluoromethylpyridin-2-yl |

[Table 1-2]

| Compound No. | A | $R^1$ | $R^2$ | m | Z-$(R^3)_n$ |
|---|---|---|---|---|---|
| I-19 | N | $CH_3$ | $CH_3$ | 1 | 5-chloropyrimidin-2-yl |
| I-20 | N | $CH_3$ | $CH_3$ | 1 | 3-nitropyridin-2-yl |
| I-21 | N | $CH_3$ | $CH_3$ | 1 | 6-chloropyridazin-3-yl |
| I-22 | N | $CH_3$ | $CH_3$ | 1 | 3-cyanopyridin-2-yl |
| I-23 | N | $CH_3$ | $CH_3$ | 1 | 6-cyanopyridin-3-yl |
| I-24 | N | $CH_3$ | $CH_3$ | 1 | 4-chloro-3-fluorophenyl |
| I-25 | N | $CH_3$ | $CH_3$ | 1 | 4-cyanophenyl |
| I-26 | N | $CH_3$ | $CH_3$ | 1 | 4-nitrophenyl |
| I-27 | N | H | H | 1 | 4-chlorophenyl |
| I-28 | N | $CH_3$ | $CH_3$ | 1 | 4-iodophenyl |
| I-29 | N | $CH_2CH_3$ | $CH_2CH_3$ | 1 | 4-chlorophenyl |
| I-30 | N | $CH_2CH_2CH_3$ | $CH_2CH_2CH_3$ | 1 | 4-chlorophenyl |
| I-31 | N | $CH_3$ | $CH_3$ | 1 | 2,4-difluorophenyl |
| I-32 | N | H | $CH_3$ | 1 | 4-chlorophenyl |
| I-33 | N | $CH_3$ | H | 1 | 4-chlorophenyl |
| I-34 | N | $CH_3$ | $CH_3$ | 1 | 4-chloro-3-hydroxyphenyl |
| I-35 | N | H | $CH_2CH_3$ | 1 | 4-chlorophenyl |
| I-36 | N | $CH_2CH_3$ | H | 1 | 4-chlorophenyl |
| I-37 | N | H | $CH_2CH_2CH_3$ | 1 | 4-chlorophenyl |
| I-38 | N | $CH_2CH_2CH_3$ | H | 1 | 4-chlorophenyl |

In the compound shown in No. I-17, a cyclopropane ring is formed of $R^1$, $R^2$, and carbon atoms to which $R^1$

and $R^2$ are bonded to each other.

The pesticidally or industrially acceptable salt of the azole derivative (I) particularly includes salts of cations or acid addition salts of acids, where the cations and anions thereof do not adversely affect the action of the azole derivative (I). Suitable cations are particularly ions of alkali metals (preferably sodium and potassium), alkaline earth metals (preferably calcium, magnesium and barium), and transition metals (preferably manganese, copper, zinc, and iron), if desired, ammonium ions which may have 1 to 4 $C_1$-$C_4$-alkyl substituents and/or one phenyl or benzyl substituent (preferably diisopropylammonium, tetramethylammonium, tetrabutylammonium, andtrimethylbenzylammonium), and furthermore, phosphonium ions, sulfonium ions (preferably tri ($C_1$-$C_4$-alkyl) sulfonium), and sulfoxysonium ions (preferably tri($C_1$-$C_4$-alkyl) sulfoxonium).

Useful acid addition salt anions are mainly chloride ions, bromide ions, fluoride ions, hydrogen sulfate ions, sulfate ions, dihydrogen phosphate ions, hydrogen phosphate ions, phosphate ions, nitrate ions, bicarbonate ions, carbonate ions, sulfonate, aromatic sulfonate, hexafluorosilicate ions, hexafluorophosphate ions, benzoate ions, and $C_1$-$C_4$-alkanoic acid anions, preferably formate ions, acetate ions, propionate ions, and butyrate ions. These can be formed by reacting the azole derivative (I) with the corresponding acid of the anion (preferably hydrochloric acid, hydrobromic acid, sulfuric acid, phosphoric acid, nitric acid, or p-toluenesulfonic acid).

Table 2 below indicates some examples of the pesticidally or industrially acceptable salt of the azole derivative (I). A, $R^1$, $R^2$ and m in Table 2 below respectively correspond to A, $R^1$ and $R^2$ and m of the above formula (I), and Z-$(R^3)_n$ in Table 2 above respectively corresponds to a structural part represented by Z and $(R^3)_n$ in the above formula (I).

[Table 2]

| Compound No. | A | $R^1$ | $R^2$ | m | Z-$(R^3)$-$_n$ | Types of salt |
|---|---|---|---|---|---|---|
| I-S1 | N | $CH_3$ | $CH_3$ | 1 | 4-chlorophenyl | Hydrochloride |
| I-S2 | N | $CH_3$ | $CH_3$ | 1 | 4-chlorophenyl | Sulfate |
| I-S3 | N | $CH_3$ | $CH_3$ | 1 | 4-chlorophenyl | Nitrate |
| I-S4 | N | $CH_3$ | $CH_3$ | 1 | 4-chlorophenyl | p-toluene sulfonate |

[2. Method for producing azole derivative]

The azole derivative (I) can be produced by any of the methods described below. Note that, in each of the methods for producing an azole derivative described below, an aspect in a case where A in general formula (I) above is N is described for convenience of explanation. However, it is readily understood that the aspect in a case where A is CH can be produced with reference to the following producing method.

$R^1$, $R^2$, $R^3$, Z, m, and n in the scheme below respectively correspond to $R^1$, $R^2$, $R^3$, Z, m, and n of the above general formula (I). In addition, compounds represented by Formula (x) (x is a number) are simply referred to as a compound (x). Note that in the reaction of the step using a known reaction mechanism, various conditions such as reagents, bases, solvents, and the like that are subjected to the reaction, and temperature are within a range that may be appropriately set by a person skilled in the art based on common general technical knowledge.

(1) Method 1 of producing azole derivative

When $R^1$ and $R^2$ are the same groups except for hydrogen, the azole derivative (I) can be produced from a compound obtained by known technologies according to a synthesis scheme 1 below.

[Chem. 3]

(Synthesis scheme 1)

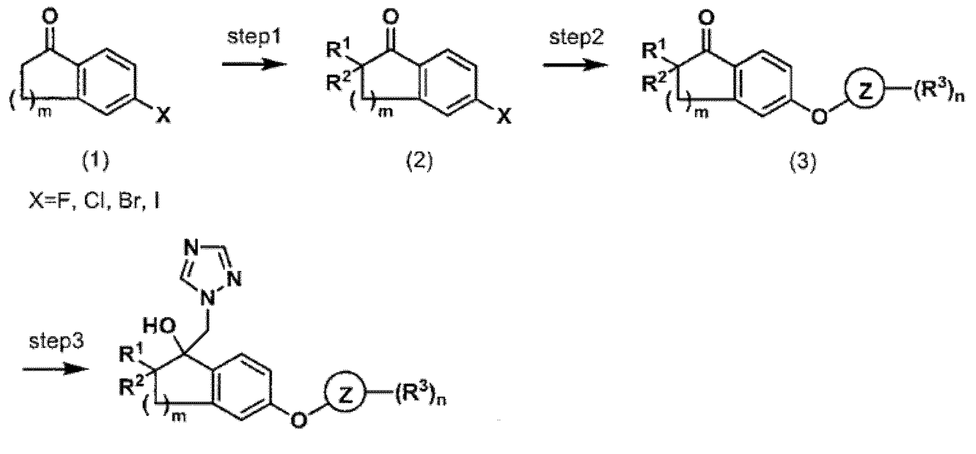


(1) (2) (3) (I)

(step 1) A compound (2) is obtained by alkylating the α-position of the ketone of a compound (1). The alkylation may be performed by a reaction using an alkylating reagent such as alkyl iodide. Examples thereof include, but are not limited to, a method for reacting at room temperature using alkyl iodide as an alkylating reagent, sodium hydride as a base, and N,N-dimethylformamide as a solvent.

(step 2) A compound (3) is obtained by substituting halogen X of the compound (2) with an aromatic heterocyclic ring having one or more $R^3$s substituted or an unsubstituted phenol or hydroxy group. The substitution in a reaction with an aromatic heterocyclic ring having a phenol or hydroxy group may vary depending on the type of X. For example, in a case where X is F or Cl, the substitution in the $S_NAr$ reaction is possible. Examples thereof include, but are not limited to, a method for reacting at 120°C using F as X, potassium carbonate as a base, and N,N-dimethylformamide as a solvent. In addition, when X is Cl, Br, or I, substitution in an Ullmann condensation reaction using a copper catalyst is possible. Note that the Ullmann condensation reaction is not limited to a reaction at high-temperature (for example, 195°C) and may be reacted using a ligand at relatively low temperature (for example, 135°C) heating conditions. Examples thereof include, but are not limited to, a method for reacting at 195°C using Br as X, copper (I) iodide as a copper catalyst, cesium carbonate as a base, and N-methylpyrrolidone as a solvent by a microwave reactor. Yet another example includes a method for adding copper (I) iodide as a copper catalyst and tris(2,4-pentanedionato) iron (III) as a ligand, and reacting the mixture using potassium carbonate as a base and N,N-dimethylformamide as the solvent, with an oil bath at 135°C heating conditions.

(step 3) A compound (3) is oxiranated by a Corey-Chaykovsky reaction followed by azolation to obtain an azole derivative (I). The oxiranation and azolation may be performed stepwise as separate reactions, but in the present embodiment, they are performed in one pot. By performing the oxiranation and azolation reactions in one pot, the number of steps can be reduced. When a one-pot reaction is performed, the target azole derivative (I) can be obtained by reacting the compound (3) with the coexistence of 1,2,4-triazole or an alkali metal salt thereof and sulfur ylide, in a solvent. Specifically, by mixing the compound (3) and 1,2,4-triazole or alkali metal salt thereof in a solvent, and intermittently adding the ylide reagent and the base to the compound (3), the intermediate product oxirane produced in the reaction system can be sequentially azolated to obtain the target azole derivative (I).

Examples of the solvent include a polar solvent with amide bonds such as N-methylpyrrolidone, N,N-dimethylacetamide, and N,N-dimethylformamide, a mixed solvent of the polar solvent and alcohol, and dimethyl sulfoxide. In addition, examples of alcohols in the mixed solvent include tert-butanol.

Examples of the sulfur ylide include sulfonium methylide, such as dimethyl sulfonium methylide, and sulfoxonium methylide, such as dimethyl sulfoxonium methylide, and the like. The sulfonium methylides or sulfoxonium methylides to be used can be produced by reacting, in solvent, an ylide reagent such as sulfonium salt (for example, trimethylsulfonium iodide and trimethylsulfonium bromide) or sulfoxonium salt (for example, trimethylsulfoxonium iodide and trimethylsulfoxonium bromide (TMSOB)) with a base. Examples of the base include a metal hydride compound such as sodium hydride, and alkali metal alkoxide such as sodium methoxide, sodium ethoxide, sodium tert-butoxide, and potassium tert-butoxide. Further, an alkali metal salt of 1,2,4-triazole may be used.

Examples of the one-pot reaction of oxiranation and azolation include, but are not limited to, a method for mixing

the compound (3) and 1,2,4-triazole sodium salt in N-methylpyrrolidone at 80°C and adding TMSOB as an ylide reagent and sodium tert-butoxide as a base, separately.

On the other hand, examples of a reaction method for performing oxiranation and azolation stepwise include, in the oxiranation, a method of reacting at room temperature using trimethylsulfonium iodide (TMSI) as an ylide reagent, sodium hydride as a base, and dimethyl sulfoxide as a solvent. In addition, in the azolation thereafter, a method for reacting at 80°C using 1,2,4-triazole, diazabicycloundecene (DBU) as a base, and dimethyl sulfoxide as a solvent.

(1') Method 1' of producing azole derivative

If X is Cl, I, or Br, instead of steps 2 and 3, as represented in a synthetic scheme 1' below, azole is introduced into the compound (2) to obtain a compound (4), and the ether skeleton is synthesized by the Ullmann condensation reaction after the introduction of azole so as to obtain the azole derivative (I).

[Chem. 4]

(Synthesis scheme 1')

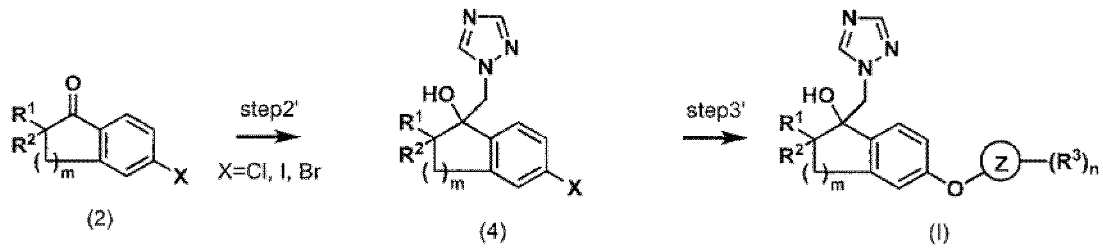


(2) (4) (I)

(step 2') A compound (4) is obtained by introducing azole into the compound (2). The azolation of the compound (2) may be performed in a similar manner as in the aforementioned step 3. Examples thereof include a method for reacting in the one pot described in the aforementioned step 3.

(step 3') The azole derivative (I) is obtained by synthesizing an ether skeleton from the compound (4) by an Ullmann condensation reaction using an aromatic heterocyclic ring substituted with one or more $R^3$ or having an unsubstituted phenol or hydroxy group. Even in the Ullmann condensation reaction here, it is not limited to reactions at high-temperature (for example, 195°C) and may be reacted using a ligand at relatively low temperature (for example, 135°C) heating conditions. Yet another Examples thereof include, but are not limited to, a method for adding copper (I) iodide as a copper catalyst and tris(2,4-pentanedionato) iron (III) as a ligand, and reacting the mixture using potassium carbonate as a base and N,N-dimethylformamide as the solvent, with an oil bath at 135°C.

(2) Method 2 of producing azole derivative

When either $R^1$ or $R^2$ is H, the azole derivative (I) can be produced from a compound obtained by known technologies according to a synthesis scheme 2 below. In the synthesis schemes 2 and 2' below, $R^1$ = H is exemplified. In addition, when $R^1$ and $R^2$ are alkyl groups which are different from each other, the azole derivative (I) can be produced from a compound (8) obtained by the synthesis scheme 2 below.

[Chemical Formula 5]

(Synthesis scheme 2)

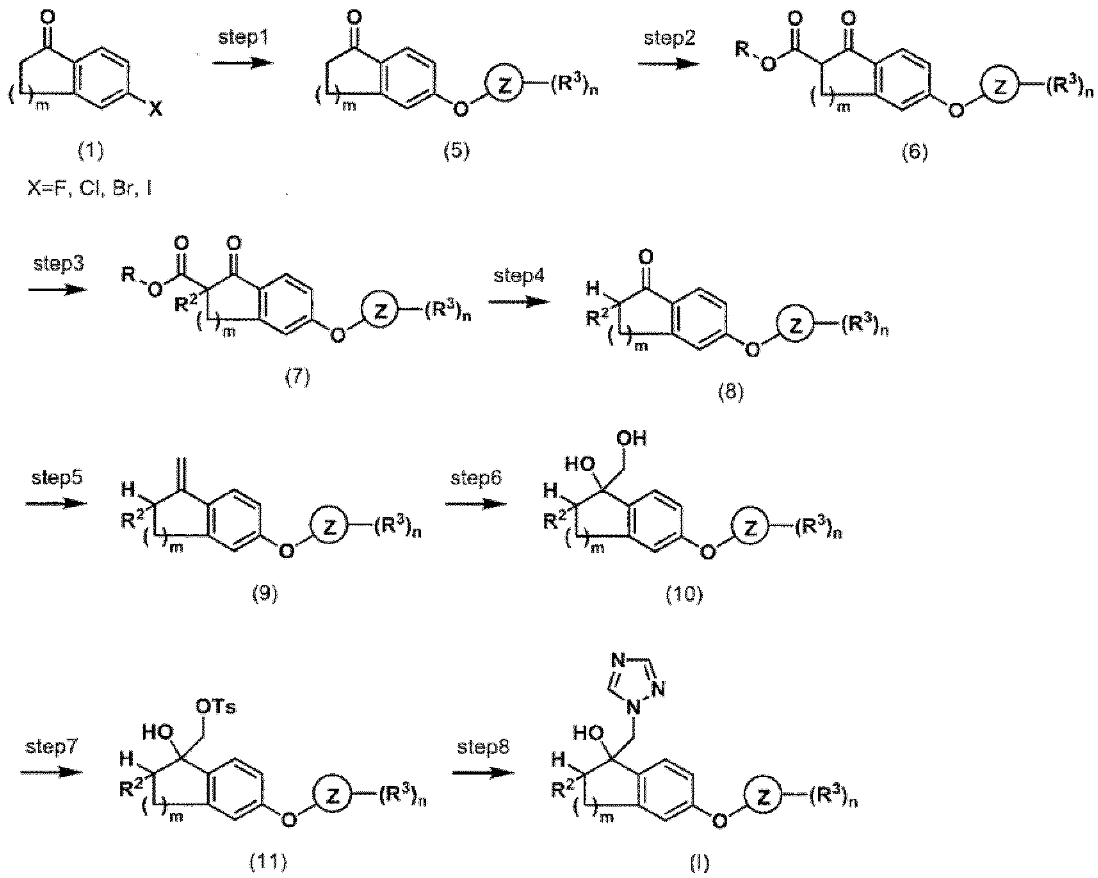


(step 1) The halogen X of compound (1) is substituted with an aromatic heterocyclic ring having a substituted or unsubstituted phenol or hydroxy group by an Ullmann reaction or an SNAr reaction to obtain compound (5) from compound (1). Note that when hydrogen is present at the ketone α-position as in the compound (1), a side reaction originating from the ketone α-position may be prioritized under basic high-temperature conditions. Therefore, in such a case, the $S_NAr$ reaction, which is a relatively mild condition, is suitable. Examples thereof include, but are not limited to, a method for reacting at 120°C using F as X, potassium carbonate as a base, and N,N-dimethylformamide as a solvent.

(step 2) A compound (6) is obtained by increasing the carbon content of the compound (5) in the form of β-ketoester. This protects one of the substituted positions and the methylene is activated against nucleophilic displacement reactions. Examples of the method for increasing the carbon content include a method of reacting dialkyl carbonate represented by ROCOOR (R is an alkyl group and two Rs may be the same or different from each other) as a reaction reagent and solvent by heating under reflux. Note that when the compound (5) is directly alkylated, since a di-substituent can also be obtained, it is difficult to selectively synthesize mono-substituent. Examples thereof include, but are not limited to, a method for reacting using dimethylcarbonate as a reaction reagent and solvent, sodium hydride as a base, by adding a small amount of methanol and heating under reflux.

(step 3) A compound (7) is obtained by alkylating active methine of the compound (6). The alkylation may be performed by a reaction using an alkylating reagent such as alkyl iodide. Examples thereof include, but are not limited to, a method for reacting at 80°C using isopropyl iodide as an alkylating reagent, sodium hydride as a base, and N,N-dimethylformamide as a solvent.

(step 4) A compound (8) is obtained by hydrolyzing and decarbonating the ester of the compound (7). Examples thereof include, but are not limited to, a method for reacting using a 30% sodium hydroxide aqueous solution as a base and tetrahydrofuran as a solvent, by heating under reflux.

(step 5) A compound (9), which is an olefin, is obtained from the compound (8), which is a ketone, by Wittig reaction. Note that when both $R^1$ and $R^2$ are H, steps 2, 3, and 4 may be omitted, and the compound (5) may be directly olefinated. Examples of the ylide reagent that becomes the phosphorus ylide include methyl triphenylphosphonium

bromide. More specific examples thereof include, but are not limited to, a method for reacting at room temperature using methyl triphenylphosphonium bromide as an ylide reagent, potassium tert-butoxide as a base, and tetrahydrofuran as a solvent.

(step 6) A compound (10), which is vic-diol, is synthesized by oxidizing the compound (9) with the coexistence of catalytic amount of osmium tetroxide and reoxidant. Examples thereof include, but are not limited to, a method for reacting at room temperature using catalytic amount of osmium tetroxide, N-methylmorpholine oxide as a reoxidizer, and a mixture of water and acetone as a solvent. Note that the azole derivative (I) may be obtained by oxiranation of the compound (9).

(step 7) A compound (11) is obtained by introducing a sulfonyl group as a leaving group into the primary hydroxy group of the compound (10). Substituted sulfonyl chlorides represented by $R^4SO_2Cl$ are used for the introduction of leaving groups. Here, $R^4$ represents an alkyl group, a phenyl group, or a naphthyl group having 1 to 3 carbon atoms, in which a hydrogen atom may be substituted. $R^4$ is preferably a 4-methyl phenyl group. Examples thereof include, but are not limited to, a method for reacting at 0°C using p-toluenesulfonyl chloride as a leaving group introduction reagent, pyridine as a base, and chloroform as a solvent.

(step 8) An azole derivative (I) is obtained by azolation of the compound (11) using an alkali metal salt of 1,2,4-triazole. Examples thereof include, but are not limited to, a method for reacting at 120°C using 1,2,4-triazole sodium salt as an azolation reagent and N-methylpyrrolidone as a solvent.

If $R^1$ and $R^2$ are alkyl groups which are different from each other, the ketone $\alpha$-position of the compound (8) may be alkylated and $R^1$ different from $R^2$ may be introduced. The alkylation may be performed by a reaction using an alkylating reagent such as alkyl iodide. Examples thereof include, but are not limited to, a method for reacting at room temperature using alkyl iodide as an alkylating reagent, sodium hydride as a base, and N,N-dimethylformamide as a solvent. By performing step 5 to step 8 after the alkylation, it is possible to obtain an azole derivative (I) in which $R^1$ and $R^2$ are alkyl groups, which are differ from each other.

(2') Method 2' of producing azole derivative

If X is Cl, I, or Br, instead of step 1, as represented in a synthetic scheme 2' below, finally, the azole derivative (I) can be obtained by substituting the halogen X of the compound (24) with an aromatic heterocyclic ring having a substituted or unsubstituted phenol or hydroxy group by the Ullmann condensation reaction. (Synthesis scheme 2')

[Chemical Formula 6]

(Synthesis scheme 2')

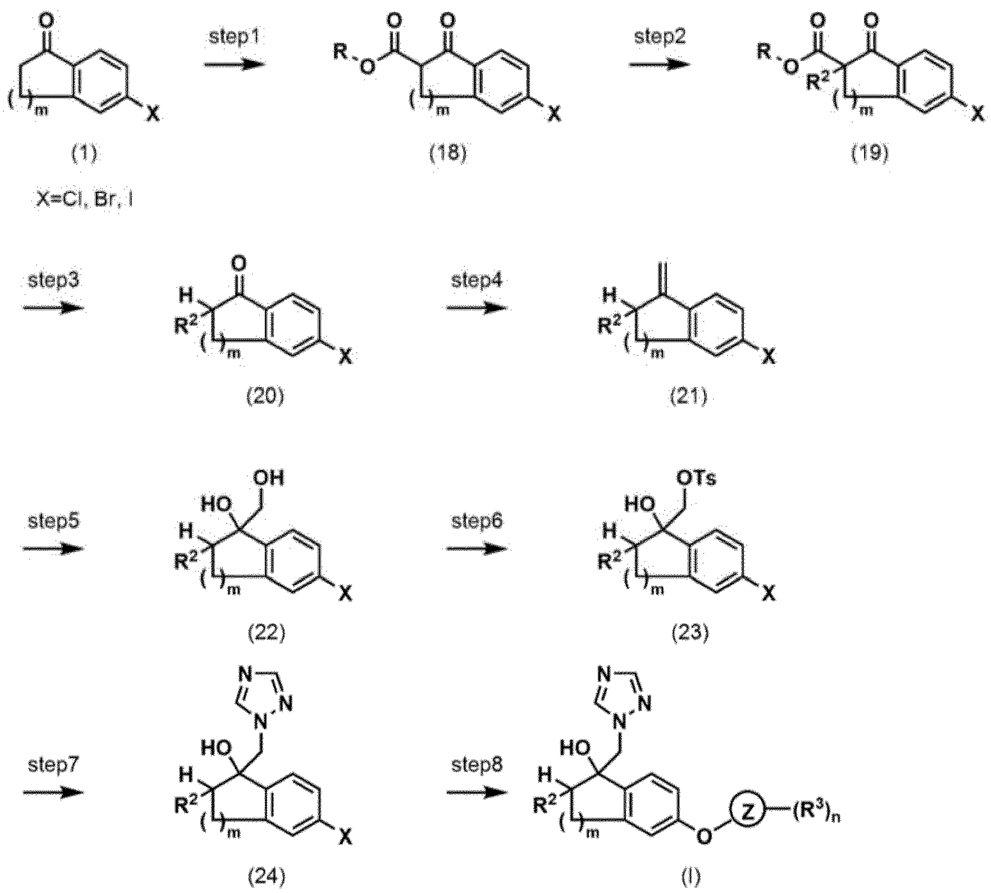


(3) Method 3 of producing azole derivative

When $R^1$ and $R^2$ are bonded together to form a cyclopropane ring with the carbon atom to which the $R^1$ and $R^2$ are bonded, the azole derivative (I) can be produced from a compound obtained by known technologies according to a synthesis scheme 3 below.

[Chemical Formula 7]

(Synthesis Scheme 3)

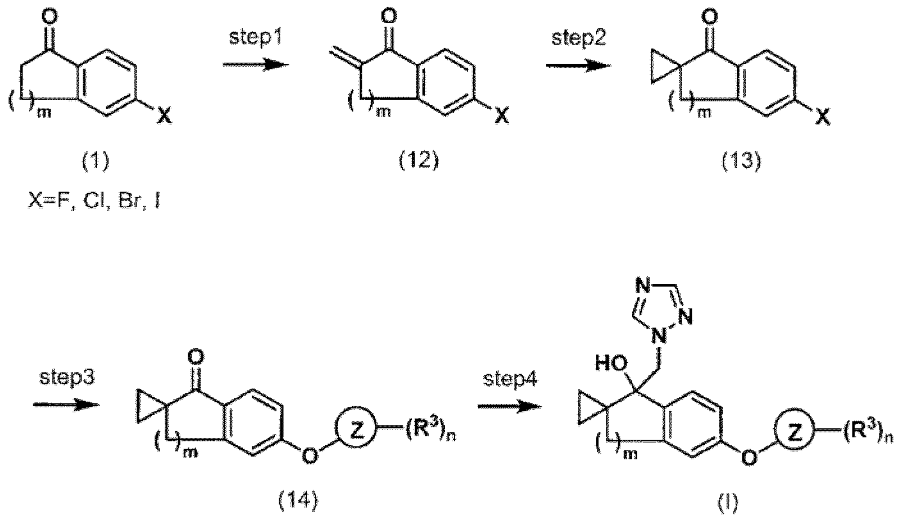


(1) X=F, Cl, Br, I

(12)

(13)

(14)

(I)

(step 1) A compound (12) is obtained by introducing methylene to a ketone $\alpha$-position of the compound (1). The method for introducing methylene can be performed with reference to the method described in Non Patent Document: Org. Syn. Coll., vol. 7 (1990), p332. Examples thereof include, but are not limited to, a method for reacting paraformaldehyde with N-methylanilinium trifluoroacetate by heating under reflux in tetrahydrofuran.

(step 2) A cyclopropane ring is introduced by allowing a sulfur ylide to act on the exomethylene at the $\alpha$-position of the ketone of the compound (12) in a Corey-Chaykovsky reaction so as to obtain a compound (13). In general, when sulfoxonium ylide is allowed to act, Michael addition occurs, but oxiranation occurs when sulfonium ylide is used. Therefore, it is preferable to use a sulfoxonium salt as the ylide reagent. Examples thereof include, but are not limited to, a method for reacting at room temperature using trimethylsulfoxonium iodide as an ylide reagent, sodium hydride as a base, and dimethyl sulfoxide as a solvent.

(step 3) A compound (14) is obtained by substituting the halogen X of the compound (13) with an aromatic heterocyclic ring having a substituted or unsubstituted phenol or hydroxy group. Specifically, this is the same as the synthesis method for the compound (3) in step 2 in the synthesis scheme 1 described above.

(step 4)A compound (14) is oxiranated by a Corey-Chaykovsky reaction followed by azolation to obtain an azole derivative (I). Specifically, this is the same as the synthesis method for the azole derivative (I) in step 3 in the synthesis scheme 1 described above.

(4) Method 4 of producing azole derivative

When Z is an aromatic heterocyclic ring, the azole derivative (I) can be produced from a compound obtained by known technologies according to a synthesis scheme 4 below.

[Chemical Formula 8]

(Synthesis Scheme 4)

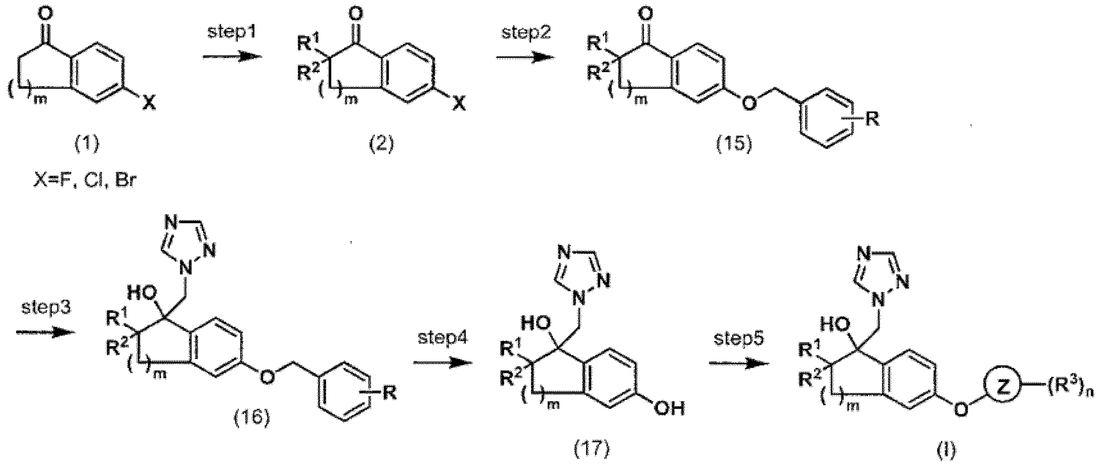


(step 1) A compound (2) is obtained by alkylating the α-position of the ketone of a compound (1). Specifically, this is the same as the synthesis method for the compound (2) in step 1 in the synthesis scheme 1 described above.

(step 2) A compound (15) is synthesized by the $S_NAr$ reaction between the compound (2) and a benzyl alcohol having a substituent R. In this case, F having high reactivity is preferable as X. Examples thereof include, but are not limited to, a method for reacting at room temperature using F as X, potassium tert-butoxide as a base, and N,N-dimethylformamide as a solvent. Note that examples of R include, but is not limited to, hydrogen, halogen, and a methoxy group.

(step 3) A compound (15) is oxiranated by a Corey-Chaykovsky reaction followed by azolation to obtain a compound (16). Specifically, this is the same as the synthesis method for the azole derivative (I) in step 3 in the synthesis scheme 1 described above.

(step 4) A compound (17) is synthesized by catalytically reducing the compound (16) under a hydrogen atmosphere using a palladium-based catalyst. Examples thereof include, but are not limited to, a method for reacting at room temperature using palladium carbon as a catalyst and ethanol as a solvent under a hydrogen atmosphere.

(step 5) The azole derivative (I) is synthesized by reacting the compound (17) with a heterocyclic ring having halogen as a nucleophile. Suitably a $S_NAr$ reaction is used. When a $S_NAr$ reaction is used, F or Cl is preferable as the halogen contained in the heterocyclic ring, and F is more preferable. Examples thereof include, but are not limited to, a method for reacting at 60°C using pyridine obtained by substituting at least one hydrogen atom with a fluorine atom, as a heterocycle having a halogen, cesium carbonate as a base, and N,N-dimethylformamide as a solvent.

[3. Method for producing N-oxide of azole derivative]

N-oxide can be prepared by treatment from azole derivative (I) according to the oxidation method in the related art. For example, the azole derivative (I) can be prepared by treating with an organic peracid (refer to WO 03/64572 or J. Med. Chem. 38 (11), 1892-903, 1995) such as meta-chloroperbenzoic acid. Alternatively, the azole derivative (I) can be prepared by treating with hydrogen peroxide which is an inorganic oxidizer (refer to J. Heterocyc. Chem. 18 (7), 1305-8, 1981) or oxone (refer to J. Am. Chem. Soc. 123 (25), 5692-5973, 2001). This oxidation can result in pure mono N-oxide or a mixture of different N-oxides. The mixture of N-oxides can be separated by known methods such as chromatography.

[4. Agricultural or horticultural chemical]

Since the azole derivative (I) has an imidazolyl group or a 1,2,4-triazolyl group, it forms an acid addition salt of inorganic acids and organic acids, or a metal complex. Therefore, it can be used as an active ingredient of agricultural or horticultural chemicals as a part of an acid addition salt and a metal complex.

(1) Plant disease controlling effect

The agricultural or horticultural chemical in the present embodiment exhibits a controlling effect against a wide

range of plant diseases.

Examples of applicable diseases include the following. Note that, in the parenthesis after each disease name, major pathogenic fungus(fungi) that causes the disease is (are) indicated. Examples thereof include Asian soybean rust (Phakopsora pachyrhizi), American soybean rust (Phakopsora meibomiae), brown spot of soybean (Septoria glycines), purple blotch of soybean (Cercospora kikuchii), Alternaria leaf spot of soybean (Alternaria sp.), anthracnose of soybean (Colletotrichum truncatum), frogeye leaf spot of soybean (Cercospora sojina), Rhizoctonia root rot of soybean (Rhizoctonia solani), Rhizoctonia foliar blight of soybean (Rhizoctonia solani), pod and stem blight of soybean (Diaporthe phaseolorum), phytophthora root and stem rot of soybean (Phytophthora sojae), anthracnose of bean (Colletotrichum lindemuthianum), phoma stem canker of rapeseed (Plenodomus lingam), stem canker of rapeseed (Plenodomus biglobosus), light leaf spot of rapeseed (Pyrenopeziza brassicae), club root of rapeseed (Plasmodiophora brassicae), Verticillium wilt of rapeseed (Verticillium longisporum), Alternaria blackspot of rapeseed (Alternaria spp.), rice blast (Pyricularia oryzae), brown spot of rice (Cochliobolus miyabeanus), bacterial leaf blight of rice (Xanthomonas oryzae pv. oryzae), rice sheath blight (Rhizoctonia solani), stem rot of rice (Helminthosporium sigmoideum), bakanae disease of rice (Gibberella fujikuroi), rice seedling blight (Pythium aphanidermatum), take-all disease of rice (Gaeumannomyces graminis), powdery mildew of barley (Blumeria graminis f. sp. hordei), barley stem rust (Puccinia graminis), yellow rust of barley (Puccinia striiformis), leaf stripe of barley (Pyrenophora graminea), scald of barley (Rhynchosporium secalis), loose smut of barley (Ustilago nuda), net blotch of barley (Pyrenophora teres), Fusarium head blight of barley (Fusarium graminearum, Microdochium nivale), powdery mildew of wheat (Erysiphe graminis f. sp. tritici), brown rust of wheat (Puccinia recondita), yellow rust of wheat (Puccinia striiformis), eyespot of wheat (Pseudocercosporella herpotrichoides), Fusarium head blight of wheat (Fusarium graminearum, Microdochium nivale), glume blotch of wheat (Phaeosphaeria nodorum), septoria leaf blotch of wheat (Zymoseptoria tritici), pink snow mold of wheat (Microdochium nivale), take-all of wheat (Gaeumannomyces graminis), wheat glume spot (Epicoccum spp.), yellow leaf spot of wheat (Pyrenophora tritici-repentis), Typhula snow blight of wheat (Typhula incarnata, Typhula ishikariensis), dollar spot of grasses (Sclerotinia homoeocarpa), grass large patch (Rhizoctonia solani), brown patch (Rhizoctonia solani), grass anthracnose (Colletotrichum graminicola), gray leaf spot of grass (Pyricularia grisea), necrotic ring spot of grass (Ophiosphaerella korrae), red thread disease of grass (Laetisaria fuciformis), grass rust (Puccinia zoysiae), summer patch of grass (Magnaporthe poae), take-all root rot of grass (Gaeumannomyces graminis), brown ring patch (Waitea circinata), fairy ring of grass (Agaricus spp., Calvatia cyathiformis, Chlorophyllum molybdite, Clitocybe spp., Lepiota app., Lepista subnuda, Lycoperdon spp., Marasmius oreades, Scleroderma spp., Tricholoma spp., and the like), pink snow mold of grass (Microdochium nivale), gray snow moldof grass (Typhula incarnate, Typhula ishikariensis), Curvularia leaf blight of grass (Curvularia sp.), Rhizoctonia patch (Ceratobasidium sp.), Zoysia decline (Gaeumannomyces sp., Phialophora sp.), corn smut (Ustilago maydis), anthracnose of corn (Colletotrichum graminicola), eyespot of corn (Kabatiella zeae), gray leaf spot of corn (Cercospora zeae-maydis), northern corn leaf blight (Setosphaeria turcica), northern corn leaf spot (Cochliobolus carbonum), brown spot of corn (Physoderma maydis), rust of corn (Puccinia spp.), southern corn leaf blight (Bipolaris maydis), yellow leaf blight of corn (Phyllosticta maydis), stalk rot of corn (Gibberella zeae), sugar cane rust (Puccinia spp.), powdery mildew of cucurbits (Sphaerotheca fuliginea), anthracnose of cucurbits (Colletotrichum lagenarium, Glomerella cingulata), cucumber downy mildew (Pseudoperonospora cubensis), cucumber damping-off (Phytophthora capsici), Fusarium wilt of cucumber (Fusarium oxysporum f. sp. cucumerinum), Fusarium wilt of watermelon (Fusarium oxysporum f. sp. niveum), apple powdery mildew (Podosphaera leucotricha), apple black spot (Venturia inaequalis), apple blossom blight (Monilinia mali), apple alternaria blotch (Alternaria alternata), apple Valsa canker (Valsa ceratosperma), pear black spot (Alternaria kikuchiana), pear powdery mildew (Phyllactinia pyri), pear rust (Gymnosporangium asiaticum), pear scab (Venturia nashicola), strawberry powdery mildew (Podosphaera aphanis ), stone fruits brown rot (Monilinia fructicola), citrus fruits blue mold (Penicillium italicum), grape powdery mildew (Uncinula necator), grape downy mildew (Plasmopara viticola), grape ripe rot (Glomerella cingulata), grape rust (Phakopsora euvitis), yellow Sigatoka of banana (Mycosphaerella musicoka), black Sigatoka of banana (Mycosphaerella fijiensis), tomato powdery mildew (Erysiphe cichoracearum), tomato early blight (Alternaria solani), eggplant powdery mildew (Erysiphe cichoracearum), potato early blight (Alternaria solani), potato anthracnose (Colletotrichum coccodes), potato powdery mildew (Erysiphe cichoracearum, Leveillula taurica), potato late blight (Phytophthora infostans), tobacco powdery mildew (Erysiphe cichoracearum), tobacco brown spot (Alternaria longipes), cercospora leaf spot of beet (Cercospora beticola), sugar beet powdery mildew (Erysiphe betae), sugar beet leaf blight (Thanatephorus cucumeris), sugar beet rood rot (Thanatephorus cucumeris), sugar beet root rot (Aphanomyces cochlioides), Fusarium wilt of radish (Fusarium oxysporum f. sp. raphani), tea anthracnose (Discula theae-sinensis), tea blister blight (Exobasidium vexans), tea brown round spot (Pseudocercospora ocellata, Cercospora chaae), tea early blight (Pestalotiopsis longiseta, Pestalotiopsis theae), tea net blister blight (Exobasidium reticulatum), cotton Alternaria leaf spot (Alternaria spp.), cotton anthracnose (Glomerella spp.), cotton Ascochyta blight (Ascochyta gossipii), cotton rust (Puccinia spp., Phakopsora gossypii), Cercospora blight and leaf spot of cotton (Cercospora spp.), Diplopia boll rot of cotton (Diplopia spp.), Hard lock of cotton (Fusarium spp.), Phoma bright of cotton (Phoma spp.), Stemphylium leaf spot of cotton (Stemphylium spp.), peanut late leaf spot (Cercosporidium personatum), peanut brown leaf spot (Cercospora arachidicola), peanut southern blight (Sclerotium

rolfsii), peanut rust (Puccinia arachidis), gray mold that affects various crops (Botrytis cinerea), diseases of Pythium (Pythium spp.), and Sclerotium disease (Sclerotinia sclerotiorum). Examples also include seed-borne diseases or early-growth diseases of various plants caused by the genus Aspergillus, the genus Cochliobolus, the genus Corticium, the genus Diplodia, the genus Penicillium, the genus Fusarium, the genus Gibberella, the genus Mucor, the genus Phoma, the genus Phomopsis, the genus Pyrenophora, the genus Pythium, the genus Rhizoctonia, the genus Rhizopus, the genus Thielaviopsis, the genus Tilletia, the genus Trichoderma, and the genus Ustilago.

The agricultural or horticultural chemical according to the present embodiment can be used as a fungicide. Furthermore, the agricultural or horticultural chemical according to the present embodiment exhibits a particularly excellent controlling effect against diseases of wheat leaf blight, wheat brown rust, and wheat powdery mildew, among the diseases described above. Accordingly, the agricultural or horticultural chemical is advantageously used to control wheat, but is not limited to such application.

The agricultural or horticultural chemical in the present embodiment can be used for all plants, and examples of applicable plants include the following; Poaceae such as rice, wheat, barley, rye, oat, triticale, corn, sorghum, sugar cane, turf, bentgrass, bermudagrass, fescue, and ryegrass; Legumes such as soybean, peanut, kidney bean, pea, adzuki bean, and alfalfa; Convolvulaceae such as sweet potato; Solanaceae such as capsicum, sweet pepper, tomato, eggplant, potato, and tobacco; Polygonaceae such as buckwheat; Asteraceae such as sunflower; Araliaceae such as ginseng; Brassicaceae such as rapeseed, Chinese cabbage, turnip, cabbage, and Japanese radish; Chenopodiaceae such as sugar beet; Malvaceae such as cotton; Rubiaceae such as coffee tree; Sterculiaceae such as cacao; Theaceae such as tea; Cucurbitaceae such as watermelon, melon, cucumber, and pumpkin; Liliaceae such as onion, leek, and garlic; Rosaceae such as strawberry, apple, almond, apricot, plum, yellow peach, Japanese plum, peach, and pear; Apiaceae such as carrot; Araceae such as taro; Larvae such as mango; Bromeliaceae such as pineapple; Caricaceae such as papaya; Ebenaceae such as persimmon; Ericaceae such as blueberry; Juglandaceae such as pecan; Musaceae such as banana; Oleaceae such as olive; Palmae such as coconut, and date; Rutaceae such as mandarin orange, orange, grapefruit, and lemon; Vitaceae such as grape; flowers and ornamental plants, trees other than fruit trees; and other ornamental plants. Other examples include wild plants, cultivars, plants and cultivars bred by known hybridizing or plasmogamy, and genetically recombined plants and cultivars obtained by gene manipulation. Examples of genetically recombined plants and cultivars include herbicide-tolerant crops, pest-resistant crops in which an insecticidal protein-producing gene has been recombined, pathogen-resistant crops in which a pathogen resistance derivative-producing gene has been recombined, taste-improved crops, yield-improved crops, preservation-improved crops, and yield-improved crops. Examples of genetically recombined cultivar that has been approved in each country include those stored in the database of the International Service for the Acquisition of Agri-biotech Applications (ISAAA). Specific examples include those containing trade names such as Roundup Ready, Liberty Link, IMI, SCS, Clearfield, Enlist, B.t., BXN, Poast Compatible, AgriSure, Genuity, Optimum, Powercore, DroughtGard, YieldGard, Herculex, WideStrike, Twinlink, VipCot, GlyTol, Newleaf, KnockOut, BiteGard, BtXtra, StarLink, Nucotn, NatureGard, Protecta, SmartStax, Power Core, InVigor, and Bollgard.

(2) Formulation

The agricultural or horticultural chemical is generally prepared by mixing azole derivative (I), which is an active ingredient, with a solid carrier or a liquid carrier (diluent), a surfactant, and other formulation aid and the like, and formulating the mixture into various forms such as a powder, a hydration agent, a granule, and an emulsion for use. These formulations are prepared so that azole derivative (I) is contained as an active ingredient in an amount of 0.1 to 95 wt.%, preferably 0.5 to 90 wt.%, and more preferably 1 to 80 wt.%.

Examples of the solid carrier, liquid carrier, and surfactant used as formulation aids are as follows. First, examples of the solid carrier include powder carriers and granular carriers such as minerals such as clay, talc, diatomaceous earth, zeolite, montmorillonite, bentonite, acid clay, activated clay, attapulgite, calcite, vermiculite, perlite, pumice, and silica sand; synthetic organic materials such as urea; salts such as calcium carbonate, sodium carbonate, sodium sulphate, slaked lime, and baking soda; synthetic inorganic materials such as amorphous silica such as white carbon and titanium dioxide; plant carriers such as wood flour, corn stalk (cob), walnut shell (nut shell), fruit core, chaff, sawdust, bran, soy flour, powdered cellulose, starch, dextrin, and sugars; and various polymeric carriers such as crosslinked lignin, cation gel, gelatin gelated by heat or a polyvalent metal salt, water-soluble polymer gel such as agar, chlorinated polyethylene, chlorinated polypropylene, polyvinyl acetate, polyvinyl chloride, ethylene-vinyl acetate copolymer, and urea-aldehyde resin.

Examples of the liquid carrier include aliphatic solvents (paraffins), aromatic solvents (for example, xylene, alkylbenzene, alkylnaphthalene, and solvent naphtha), mixed solvents (kerosene), machine oils (refined high-boiling aliphatic hydrocarbons), alcohols (for example, methanol, ethanol, isopropanol, and cyclohexanol), polyhydric alcohols (for example, ethylene glycol, diethylene glycol, propylene glycol, hexylene glycol, polyethylene glycol, and polypropylene glycol), polyhydric alcohol derivatives (for example, propylene glycol ether), ketones (for example, acetone, acetophenone, cyclohexanone, methylcyclohexanone, and γ-butyrolactone), esters (fatty acid methyl ester (coconut oil fatty acid methyl ester), ethylhexyl lactate, propylene carbonate, dibasic acid methyl ester (succinic acid dimethyl ester, glutamic acid dimethyl ester, and adipic acid dimethyl ester)), nitrogen-containing carriers (N-alkylpyrrolidones), oils and fats (for example, coconut oil, soybean oil, and rapeseed oil), amide solvents [dimethylformamide, (N,N-dimethyloctaneamide, N,N-dimethyldecaneamide, 5-(dimethylamino)-2-methyl-5-oxo-valeric acid methyl ester, N-acylmorpholine-based solvents (for example, CAS NO. 887947-29-7)], dimethyl sulfoxide, acetonitrile, and water.

Examples of the nonionic surfactants include sorbitan fatty acid ester, polyoxyethylene sorbitan fatty acid ester, sucrose fatty acid ester, polyoxyethylene fatty acid ester, polyoxyethylene resin acid ester, polyoxyethylene fatty acid diester, polyoxyethylene alkyl ether, polyoxyethylene alkylphenyl ether, polyoxyethylene dialkyl phenyl ether, polyoxyethylene alkyl phenyl ether formalin condensate, polyoxyethylene/polyoxypropylene block polymer, alkyl polyoxyethylene/polyoxypropylene block polymer ether, polyoxyethylene alkylamine, polyoxyethylene fatty acid amide, polyoxyethylene fatty acid bisphenyl ether, polyoxyethylene benzylphenyl (or phenylphenyl) ether, polyoxyethylene styrylphenyl (or phenylphenyl) ether, polyoxyethylene ether and ester type silicone and fluorosurfactants, polyoxyethylene castor oil, polyoxyethylene hydrogenated castor oil, and alkyl glycosides. Examples of the anionic surfactants include salts of sulphates such as alkyl sulphate, polyoxyethylene alkyl ether sulphate, polyoxyethylene alkylphenyl ether sulphate, polyoxyethylene benzyl (or styryl) phenyl (or phenylphenyl) ether sulphate, polyoxyethylene, polyoxypropylene block polymer sulphate; salts of sulfonates such as paraffin (alkane) sulfonate, α-olefin sulfonate, dialkyl sulfosuccinate, alkylbenzene sulfonate, mono- or dialkyl naphthalene sulfonate, naphthalene sulfonate-formalin condensate, alkyl diphenyl ether disulfonate, lignin sulfonate, polyoxyethylene alkyl phenyl ether sulfonate, and polyoxyethylene alkyl ether sulfosuccinic acid half ester; salts of fatty acid such as fatty acids, N-methyl-fatty acid sarcosinates, and resin acids; salts of phosphates such as polyoxyethylene alkyl ether phosphate, polyoxyethylene mono- or dialkyl phenyl ether phosphate, polyoxyethylene benzyl (or styryl) phenyl (or phenylphenyl) ether phosphate, polyoxyethylene/polyoxypropylene block polymer, phosphatidylcholine phosphatidylethanolimine (lecithin), and alkyl phosphates. Examples of the cationic surfactants can include ammonium salts such as alkyltrimethylammonium chloride, methylpolyoxyethylene alkylammonium chloride, alkyl N-methylpyridinium bromide, mono- or dialkylmethylated ammonium chloride, alkylpentamethylpropylenediamine dichloride; and benzalkonium salts such as alkyldimethylbenzalkonium chloride, and benzethonium chloride (octylphenoxyethoxyethyl dimethylbenzylammonium chloride). The surfactant may be a biosurfactant. Examples of the biosurfactant can include rhamnolipids, surfactins, cellobiose lipids, sophorolipids, mannosyl alditol lipids, trehalose lipids, glucose lipids, oligosaccharide fatty acid esters, serauthin, lykensin, arsulofactin, spiculesporic acid, corynomycolic acid, agaritic acid, and emalzan.

Examples of the other formulation aid include inorganic salts used as pH adjusters such as sodium and potassium; fluorine-based and silicon-based defoamers; water-soluble salts such as common salt; water-soluble polymers used as thickeners such as xanthan gum, guar gum, carboxymethyl cellulose, polyvinylpyrrolidone, carboxyvinyl polymer, acrylic polymer, polyvinyl alcohol, starch derivatives and polysaccharides; alginic acid and salts thereof; metal stearates, sodium tripolyphosphate, sodium hexametaphosphate used as disintegrating dispersants; antiseptics; colorants; antioxidants; UV absorbers; and chemical damage reducers.

Some formulations are used as they are and some are diluted with a diluent such as water to a predetermined concentration before use. The concentration of azole derivative (I) when diluted before use is preferably in the range from 0.001 to 1.0%.

The amount of azole derivative (I) used is from 20 to 5000 g, and more preferably from 50 to 2000 g per 1 ha of agricultural or horticultural area such as fields, rice fields, orchards, and greenhouses. Since these concentration and quantity used differ depending on the form of the agent, time of use, usage method, usage location, target crops and the like, they may be increased or decreased within the above ranges.

(3) Other active ingredients

The agricultural or horticultural chemical in the present embodiment may be used in combination with other known active ingredients to enhance the performance as an agricultural or horticultural chemical. Examples of the other known active ingredients include known active ingredients contained in fungicides, insecticides, miticides, nematicides, herbicides, and plant growth regulators.

(3-1) Active ingredients for fungicidal use

Examples of effective components suitable for fungicidal use include sterol biosynthesis inhibitor compounds, benzimidazole compounds, succinate dehydrogenase inhibitor compounds (SDHI compounds), strobilurin-based compounds, phenylamide compounds, dicarboximide compounds, anilinopyrimidine compounds, multi-point compounds, antibiotics, carbamate compounds, quinoline compounds, organophosphorus compounds, and carboxyamide compounds.

Examples of the sterol biosynthesis inhibitor compounds include azaconazole, bitertanol, bromuconazole, difenoconazole, cyproconazole, diniconazole, fenbuconazole, fluquinconazole, fltriafol, hexaconazole, imazalil, imibenconazole, metconazole, ipconazole, myclobutanil, pefurazoate, penconazole, prochloraz, propiconazole, prothioconazole, epoxiconazole, simeconazole, tebuconazole, tetraconazole, triadimefon, triadimenol, triflumizole, triticonazole, flusilazole, oxpoconazole, mefentrifluconazole, ipfentrifluconazole, 1-((1H-1,2,4-triazole-1-yl)methyl)-5-(4-chlorobenzyl)-2-(chloromethyl)-2-methylcyclopentan-1-ol, methyl-2-((1H-1,2,4-triazole-1-yl)methyl)-3-(4-chlorobenzyl)-2-hydroxy-1-methylcyclopentane-1-carboxylate, fenpropimorph, fenpropidin, spiroxamine, tridemorph, fenarimol, pyrifenox, pyrisoxazole, nuarimol, etaconazole, piperalin, naftifine, fenpyrazamine, fenhexamid, terbinafine, aldimorph, dodemorph, pyributicarb, and triforine.

Examples of the benzimidazole compounds include carbendazim, benomyl, thiabendazole, and fuberidazole.

Examples of the succinate dehydrogenase inhibitor compounds (SDHI compounds) include bixafen, benzovindiflupyr, boscalid, fluopyram, fenfuram, flutolanil, fluxapyroxad, furametopyr, isofetamid, isopyrazam, mepronil, penflufen, penthiopyrad, sedaxane, thifluzamide, fluindapyr, pyraziflumid, pydiflumetofen, benodanil, carboxin, pyrapropoyne, inpyrfluxam, isoflucypram, inpyrfluxam, and oxycarboxin.

Examples of the strobilurin compounds include azoxystrobin, dimoxystrobin, enestrobin, fenaminstrobin, fluoxastrobin, kresoxim methyl, metominostrobin, orysastrobin, picoxystrobin, pyraclostrobin, trifloxystrobin, mandestrobin, pyribencarb, pyraoxystrobin, pyrametostrobin, flufenoxystrobin, enoxastrobin, coumoxystrobin, triclopyricarb, fenaminstrobin, and metyltetraprole.

Examples of the phenylamide compounds include benalaxyl, benalaxyl M or chiralaxyl, metalaxyl, metalaxyl M or mefenoxam, furalaxyl, and oxadixyl.

Examples of the dicarboximide compounds include procymidone, iprodione, and vinclozolin.

Examples of the anilinopyrimidine compounds include cyprodinil, mepanipyrim, and pyrimethanil.

Examples of the multi-point compounds include manzeb, maneb, metiram, propineb, thiram (thiuram), zineb, ziram, amobam, anilazine, dithianon, fluazinam, dichlofluanid, tolylfluanid, dodine, guazatine, iminoctadine (iminoctadine acetate and iminoctadine albesilate), copper, copper compounds (for example, basic copper chloride, cupric hydroxide, basic copper sulphate, copper sulphate, organic copper (oxine copper), copper nonylphenol sulfonate, and DBEDC), hydrogen carbonate (sodium hydrogen carbonate and potassium hydrogen carbonate), metallic silver, fentin, sulfur, mineral oil, baking soda, potassium carbonate, ferbum, captan, captafol, fluoroimide, chinomethionat (quinomethionate), methasulfocarb, dipymetitrone, chlorothalonil (TPN), and folpet.

Examples of the antibiotics include kasugamycin, polyoxin, streptomycin, validamycin, and oxytetracycline.

Examples of the carbamate compounds include benthiavalicarb (benthiavalicarb-isopropyl), diethofencarb, iprovalicarb, propamocarb, iodocarb, prothiocarb, and tolprocarb.

Examples of the quinoline compound include oxolinic acid, pyroquilon, quinoxyfen, and tebufloquin.

Examples of the organophosphorus compounds include dinocap, edifenphos (EDDP), fosetyl (fosetyl-aluminum, fosetyl-potassium, and fosetyl-sodium), iprobenfos (IBP), pyrazophos, meptyldinocap, and tolclofos-methyl.

Examples of the carboxyamide compounds include carpropamid, ethaboxam, fenoxanil, silthiofam, tiadinil, and isotianil.

Examples of other compounds for fungicidal use include thiophanate, thiophanate methyl, pencycuron, ametoctradin, amisulbrom, cyazofamid, cyflufenamid, cymoxanil, diclocymet, diclomezine, famoxadone, fenamidone, fenitropan, fludioxonil, fluopicolide, flusulfamide, flutianil, harpin, isoprothiolane, mandipropamid, phenamacril, metrafenone, oxathiapiprolin, fthalide, proquinazid, valifenalate, zoxamide, dichlobentiazox, fenpicoxamid, picarbutrazox, quinofumelin, dimethomorph, flumorph, pyrimorph, ferimzone, acibenzolar (acibenzolar-S-methyl), etridiazole, hymexazole, probenazole, tricyclazole, tecloftalam, hydroxyisoxazole, pyriofenone, diflumetorim, tolfenpyrad, fenazaquin, aminopyrifene, chloroinconazide, pyridachlometyl, ipflufenoquin, fluopimomide, florylpicoxamide, fluoxapiprolin, binapacryl, fentin acetate, fentin chloride, fentin hydroxide, ofurace, bupirimate, dimethirimol, ethirimol, octhilinone, chlozolinate, dimethachlone, fenpiclonil, blasticidin, triazoxide, dodine, kinoprol, biphenyl, chloroneb, dicloran, quintozene (PCNB), tecnazene (TCNB), natamycin (pimaricin), laminarin, flubeneteram, phosphoric acid, phosphate, shiitake mycelium extract, Melaleuca alternifolia (tea tree) extract, plant oils (eugenol, geraniol, and thymol), Reynoutria sachalinensis (giant knotweed) extract, cell wall of Saccharomyces cerevisiae strain LAS117, and biological pesticides (Agrobacterium radiobactor, Pseudomonas fluorescens, Pseudomonas rhodesia, Pseudomonas chlororaphis strain AFS009, Bacillus subtilis, Bacillus simplex, Bacillus amyloliquefaciens, Bacillus mycoides, nonpathogenic Erwinia carotovora, Lactobacillus plantarum, Variovorax paradoxus, Swinglea glutinosa extract, Trichoderma atroviride strain I-1237, Trichoderma atroviride strain LU132, Trichoderma atroviride strain SC1, Trichoderma asperellum strain T34, Gliocladium catenulatum or Clonostachys rosea, Streptomyces griseovirides strain K61, and Streptomyces lydicus strain WYEC108).

(3-2) Active ingredients for insecticidal use

Examples of effective components suitable for insecticidal use include organophosphorus compounds, carbamate compounds, pyrethroid compounds, nereistoxin compounds, neonicotinoid compounds, benzoylurea compounds, other insect growth control compounds, organic chlorine compounds, and compounds derived from natural products.

Examples of the organophosphorus compounds include acephate, azamethiphos, azinphos-methyl, azinphos-ethyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos (DDVP), dicrotophos, dimethoate, dimethylvinphos, disulfoton, ethion, ethoprophos, EPN, famphur, fenamiphos, fenitrothion (MEP), fenthion (MPP), fosthiazate, heptenophos, imicyafos, isofenphos, isopropyl-O-(methoxy aminothio-phosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, sulfotep, quinalphos, tebupirimfos, terbufos, tetrachlorvinphos, thiometon, triazophos, vamidothion, and trichlorfon (DEP).

Examples of the carbamate compounds include alanycarb, aldicarb, bendiocarb, benfuracarb, butocarboxim, butoxycarboxim, carbaryl (NAC), carbofuran, carbosulfan, formetanate, isoprocarb (MIPC), methiocarb, methomyl, oxamyl, pirimicarb, propoxur, thiodicarb, thiofanox, triazamate, trimethacarb, XMC, ethiofencarb, fenobucarb (BPMC), fenothiocarb, furathiocarb, metolcarb, and xylylcarb.

Examples of the pyrethroid compounds include acrinathrin, allethrin, cypermethrin, bifenthrin, bioallethrin, bioallethrin s-cyclopenthyl isomer, bioresmethrin, cycloprothrin, cyfluthrin, deltamethrin, empenthrin, dimefluthrin, esfenvalerate, etofenprox, fenpropathrin, fenvalerate, flubrocythrinate, flucythrinate, flumethrin, fluvalinate, halfenprox, cyhalothrin, metofluthrin, momfluorothrin, permethrin, prallethrin, pyrethrins or pyrethrum, resmethrin, profluthrin, tefluthrin, tetramethrin, tralomethrin, transfluthrin, imiprothrin, kadethrin, chloroprallethrin, epsilon-metofluthrin, epsilon-momfluorothrin, and cyphenothrin.

Examples of the nereistoxin compounds include cartap, bensultap, thiocyclam, thiosultap-sodium (bisultap), and monosultap.

Examples of the neonicotinoid compounds include acetamiprid, clothianidin, dinotefuran, imidacloprid, nitenpyram, thiacloprid, thiamethoxam, and nicotine.

Examples of the benzoylurea compound include bistrifluron, chlorfluazuron, diflubenzuron, flucycloxuron, flufenoxuron, hexaflumuron, lufenuron, novaluron, noviflumuron, teflubenzuron, and triflumuron.

Examples of other insect growth control compounds include buprofezin, chromafenozide, cyromazine, halofenozide, methoxyfenozide, fenoxycarb, tebufenozide, and pyriproxyfen.

Examples of the organic chlorine compounds include chlordane, aldrin, dieldrin, endosulfan, methoxychlor, lindane, and DDT.

Examples of the compounds derived from natural products include abamectin, live spores and produced crystal toxins derived from Bacillus thuringiensis, and mixtures thereof, Bacillus sphaericus, emamectin benzoate, lepimectin, milbemectin, spinetoram, spinosad, machine oil, starch, saccharified reduced starch, rapeseed oil, sodium oleate, fatty acid monoesters with glycerol or propanediol, and ferric phosphate.

Examples of other compounds for insecticidal use include chlorantraniliprole, tetrachlorantraniliprole, chlorfenapyr, cyantraniliprole, diafenthiuron, ethiprole, fipronil, flonicamid, flubendiamide, fluensulfone, flupyradifurone, indoxacarb, metaflumizone, metaldehyde, pymetrozine, pyridalyl, pyrifluquinazon, silafluofen, spirotetramat, sulfoxaflor, tolfenpyrad, afidopyropen, broflanilide, cyclaniliprole, dicloromezotiaz, flometoquin, fluazaindolizine, fluhexafon, fluxametamid, pyriprole, tetraniliprole, triflumezopyrim, hydroprene, kinoprene, methoprene, tyclopyrazoflor, flupyrimin, spiropidion, benzpyrimoxan, cyhalodiamide, isocycloseram, DNOC, sulfluramid, rotenone, nicofluprole, dimpropyridaz, methyl bromide, chloropicrin, cryolite, sulfuryl fluoride, borax, boric acid, sodium octaborate, sodium metaborate, tartar emetic, dazomet, metam, aluminium phosphide, calcium phosphide, phosphine, zinc phosphide, calcium cyanide, potassium cyanide, sodium cyanide, cyproflanilide, Cydia pomonella GV, Thaumatotibia leucotreta GV, Anticarsia gemmatalis MNPV, Helicoverpa armigera NPV, GS-omega/kappa HXTX-Hv1a peptide, azadirachtin, dicofol, lime sulfur, manzeb (mancozeb), Burkholderia spp., Wolbachia pipientis (zap), Chenopodium ambrosioides near ambrosioides extract, neem oil, Beauveria bassiana, Metarhizium anisopliae, Paecilomyces fumosoroseus, and Diatomaceous earth.

(3-3) Active ingredients for acaricidal use

Examples of active ingredients suitable for acaricidal use (acaricidal active ingredient) include acequinocyl, hydramethylnon, amidoflumet, amitraz, azocyclotin, bifenazate, bromopropylate (phenisobromolate), chlorfenson, chinomethionat, benzoximate, clofentezine, cyenopyrafen, cyflumetofen, cyhexatin, diflovidazin, dienochlor, etoxazole, fenazaquin, fenbutatin oxide, fenpyroximate, fluacrypyrim, hexythiazox, propargit (BPPS), pyflubumid, pyridaben, pyrimidifen, spirodiclofen, spiromesifen, tebufenpyrad, tetradifon, acynonapyr, cyetpyrafen, flupentiofenox, and blended oils.

(3-4) Active ingredients for nematicidal use

Examples of the most suitable active ingredient for nematicidal use (nematicidal active ingredient) include D-D (1,3-dichloropropene), DCIP (dichlorodiisopropyl ether), methyl isothiocyanate, metam sodium, cadusafos, fosthiazate, imicyafos, morantel tartrate, levamisole hydrochloride, nemadectin, cyclobutrifluram, and tioxazafen.

(3-5) Active ingredients for plant growth regulator use

Examples of the optimal active ingredients of plant growth regulators include aminoethoxyvinylglycine, chlormequat, chlorpropham, cyclanilide, dikegulac, daminozide, ethephon, flurprimidol, flumetralin, forchlorfenuron, gibberellin, salts of maleic hydrazide, mepiquat chloride, methylcyclopropene, benzylaminopurine, paclobutrazol, prohexadione, thidiazuron, tribufos (tributyl phosphorotrithioate), trinexapac-ethyl, and uniconazole.

(4) Method for controlling plant diseases

The agricultural or horticultural chemical in the present embodiment may be used, for example, in cultivated lands such as fields, paddy fields, lawns, and orchards or non-cultivated lands. In addition, the agricultural or horticultural chemical in the present embodiment can be used not only in foliage treatment such as foliage spraying but also in non-foliage treatment such as seed treatment including treatment of bulbs and tubers, soil-drenching treatment, and water surface treatment. Therefore, the method for controlling plant diseases of the present embodiment includes a step of performing foliage treatment or non-foliage treatment using the agricultural or horticultural chemical described above. When non-foliage treatment is performed, the amount of labor required can be reduced in comparison to when foliage treatment is performed.

In the application by seed treatment, the agent is attached to the seed by mixing a hydration agent, a powder, and the like with the seed and stirring, or by immersing the seed in a diluted hydration agent and the like. The seed treatment also includes seed coating treatment. The amount of active ingredients used in the case of seed treatment is, for example, from 0.01 to 10000 g, and preferably from 0.1 to 1000 g per 100 kg of seeds. The seeds treated with an agricultural and horticultural chemical may be used in the same manner as ordinary seeds.

In the case of application by irrigation treatment, a planting hole or the vicinity thereof may be treated with granules or the like at the time of the transplantation of seedling or the like, or seeds or the earth around a plant may be treated with granules, a wettable powder, or the like. The amount of active ingredients used in the case of soil-drenching treatment is, for example, from 0.01 to 10,000 g and preferably from 0.1 to 1,000 g per 1 $m^2$ of agricultural or horticultural area.

In the case of application by water surface treatment, the water surface of a paddy field may be treated with granules or the like. The amount of active ingredients used in the case of water surface treatment is, for example, from 0.1 to 10000 g and preferably from 1 to 1000 g per 10 a of the paddy field.

The amount of active ingredients used for foliar spraying is, for example, from 20 to 5000 g and preferably from 50 to 2000 g per 1 ha of the agricultural or horticultural area such as a field, a rice paddy, an orchard, and a greenhouse.

Additionally, since the concentration and quantity used differ depending on the form of the agent, time of use, usage method, usage location, target crops and the like, they may be increased or decreased within the above ranges.

[5. Industrial material protectants]

(1) Industrial material protection effect

The azole derivative (I) exhibits an excellent effect in protecting materials from a wide variety of harmful microorganisms that erode industrial materials, and thus can be also used for industrial material protectants. Examples of such microorganisms include the microorganisms listed below.

Examples thereof include aspergillus (Aspergillus sp.), trichoderma (Trichoderma sp.), penicillium (Penicillium sp.), geotrichum (Geotrichum sp.), chaetomium (Chaetomium sp.), cadophora (Cadophora sp.), ceratostomella (Ceratostomella sp.), cladosporium (Cladosporium sp.), corticium (Corticium sp.), lentinus (Lentinus sp.), lenzites (Lenzites sp.), phoma (Phoma sp.), polysticus (Polysticus sp.), pullularia (Pullularia sp.), stereum (Stereum sp.), trichosporium (Trichosporium sp.), aerobacter (Aerobacter sp.), bacillus (Bacillus sp.), desulfovibrio (Desulfovibrio sp.), pseudomonas (Pseudomonas sp.), flavobacterium (Flavobacterium sp.), and micrococcus (Micrococcus sp.), which are paper/pulp-degrading microorganisms (including slime-forming bacteria); aspergillus (Aspergillus sp.), penicillium (Penicillium sp.), chaetomium (Chaetomium sp.), myrothecium (Myrothecium sp.), curvularia (Curvularia sp.), gliomastix (Gliomastix sp.), memnoniella (Memnoniella sp.), sarcopodium (Sarcopodium sp.), stschybotrys (Stschybotrys sp.), stemphylium (Stemphylium sp.), zygorhynchus (Zygorhynchus sp.), bacillus (Bacillus sp.), and staphylococcus (Staphylococcus sp.), which

are fiber-degrading microorganisms; Fomitopsis palustris, Coriolus versicolor, aspergillus (Aspergillus sp.), penicillium (Penicillium sp.), rhizopus (Rhizopus sp.), aureobasidium (Aureobasidium sp.), gliocladium (Gliocladium sp.), cladosporium (Cladosporium sp.), chaetomium (Chaetomium sp.), and trichoderma (Trichoderma sp.), which are wood-rotting microorganisms; aspergillus (Aspergillus sp.), penicillium (Penicillium sp.), chaetomium (Chaetomium sp.), cladosporium (Cladosporium sp.), mucor (Mucor sp.), paecilomyces (Paecilomyces sp.), pilobolus (Pilobolus sp.), pullularia (Pullularia sp.), trichosporon (Trichosporon sp.), and trichothecium (Trichothecium sp.), which are leather-deteriorating microorganisms; aspergillus (Aspergillus sp.), penicillium (Penicillium sp.), rhizopus (Rhizopus sp.), trichoderma (Trichoderma sp.), chaetomium (Chaetomium sp.), myrothecium (Myrothecium sp.), streptomyces (Streptomyces sp.), pseudomonas (Pseudomonas sp.), bacillus (Bacillus sp.), micrococcus (Micrococcus sp.), serratia (Serratia sp.), margarinomyces (Margarinomyces sp.), and monascus (Monascus sp.), which are rubber-plastic degrading microorganisms; aspergillus (Aspergillus sp.), penicillium (Penicillium sp.), cladosporium (Cladosporium sp.), aureobasidium (Aureobasidium sp.), gliocladium (Gliocladium sp.), botryodiplodia (Botryodiplodia sp.), macrosporium (Macrosporium sp.), monilinia (Monilinia sp.), phoma (Phoma sp.), pullularia (Pullularia sp.), sporotrichum (Sporotrichum sp.), trichoderma (Trichoderma sp.), bacillus {(bacillus sp.), proteus (Proteus sp.), pseudomonas (Pseudomonas sp.), and serratia (Serratia sp.), which are paint-deteriorating microorganisms.

(2) Formulation

An industrial material protectant containing an azole derivative (I) as an active ingredient may also contain various components in addition to the azole derivative (I). An industrial material protectant containing the azole derivative (I) as an active ingredient can be dissolved or dispersed in an appropriate liquid carrier or mixed with a solid carrier. The industrial material protectant containing the azole derivative (I) as an active ingredient may further contain an emulsifier, a dispersant, a spreading agent, a penetration agent, a wetting agent, or a stabilizer. Furthermore, examples of the agent type of the industrial material protectant containing the azole derivative (I) as an active ingredient include wettable powder, powders, granules, tablets, pastes, suspending agents, and spraying materials. An industrial material protectant containing the azole derivative (I) as an active ingredient may include other fungicides, insecticides, or antidegradants, and the like.

The liquid carrier is not particularly limited provided that it does not react with the active ingredient. Examples of the liquid carrier include water, alcohols (for example, methyl alcohol, ethyl alcohol, ethylene glycol, and cellosolve), ketones (for example, acetone and methyl ethyl ketone), ethers (for example, dimethyl ether, diethyl ether, dioxane, and tetrahydrofuran), aromatic hydrocarbons (for example, benzene, toluene, xylene, and methyl naphthalene), aliphatic hydrocarbons (for example, gasoline, kerosene, lamp oil, mechanical oil, and fuel oil), acid amides (for example, dimethylformamide and N-methylpyrrolidone), halogenated hydrocarbons (for example, chloroform and carbon tetrachloride), esters (for example, ethyl acetate esters and glycerol esters of fatty acids), nitriles (for example, acetonitrile), and dimethyl sulfoxide.

Furthermore, fine powders or granules such as kaolin clay, bentonite, acid clay, pyrophyllite, talc, diatomaceous earth, calcite, urea, ammonium sulfate, and the like can be used as the solid carrier.

Soaps, alkyl sulfonic acids, alkyl aryl sulfonic acids, dialkyl sulfosuccinates, quaternary ammonium salts, oxyalkylamines, fatty acid esters, and polyalkylene oxide-based and anhydrosorbitol-based surfactants can be used as the emulsifier and dispersant.

When the azole derivative (I) is contained as an active ingredient in the formulation, the content ratio thereof depends on the type of agent and the purpose of use, and the content ratio may be, 0.1 to 99.9% wt.% with respect to the total mass of the formulation. Note that in actual use, the treatment concentration is preferably adjusted by adding solvents, diluents, extenders, and the like as appropriate so that the level of treatment is generally from 0.005 to 5 wt.%, and preferably from 0.01 to 1 wt.%.

As described above, the azole derivative (I) exhibits excellent fungicidal activity against many types of fungi that cause plant diseases. That is, the agricultural or horticultural chemical containing the azole derivative (I) as an active ingredient has low toxicity toward human and animals and excellent safety in handling, and can exhibit a high controlling effect against a wide variety of plant diseases.

[Summary]

As described above, the azole derivative according to an embodiment of the present invention is an azole derivative represented by general formula (I) above, or an N-oxide or acceptable salt thereof.

[In Formula (I), A is N or CH;

$R^1$ and $R^2$ are each independently hydrogen, a $C_1$-$C_6$-alkyl group, a $C_3$-$C_8$-cycloalkyl group, or a $C_3$-$C_8$-cycloalkyl-$C_1$-$C_4$-alkyl group;

$R^1$ and $R^2$ may be bonded to each other to form a ring;
Z is a phenyl group or a 5- or 6-membered aromatic heterocyclic ring containing 1, 2, 3, or 4 heteroatoms selected from O, N, and S;
$R^3$ is halogen, a hydroxy group, an amino group, a nitrile group, a nitro group, a pentafluorosulfanyl group, a $C_1$-$C_4$-alkyl group, a $C_1$-$C_4$-haloalkyl group, a $C_1$-$C_4$-alkoxy group, or a $C_1$-$C_4$-haloalkoxy group;
$R^3$ is bonded to any substitution position of Z in a number of n;
n is 0, 1, 2, 3, 4, or 5; and
m is 1 or 2].

In addition, a preferable azole derivative (I) according to an embodiment of the present invention is an azole derivative (I) in which $R^1$ and $R^2$ are each independently hydrogen, a $C_1$-$C_6$-alkyl group, a $C_3$-$C_8$-cycloalkyl group, or a $C_3$-$C_8$-cycloalkyl-$C_1$-$C_4$-alkyl group, and in which $R^1$ and $R^2$ may be bonded to form a ring.

In addition, in the azole derivative according to an embodiment of the present invention, in the general formula (I) above, m is preferably 1.

In addition, in azole derivative according to an embodiment of the present invention, in the general formula (I) above, Z is preferably a phenyl group.

The method for producing an azole derivative according to an embodiment of the present invention is a method of producing the azole derivative described above by reacting the compound represented by general formula (II) below with the coexistence of 1,2,4-triazole or imidazole or an alkali metal salt thereof and sulfur ylide.

[Chem. 9]

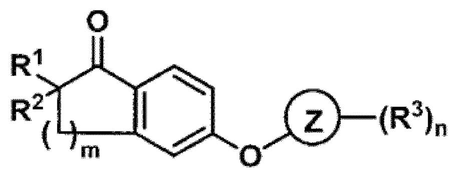

(I I)

wherein, $R^1$, $R^2$, $R^3$, Z, m, and n are the same as $R^1$, $R^2$, $R^3$, Z, m, and n in formula (I) respectively.
Furthermore, the agricultural or horticultural chemicals or industrial material protectants containing the azole derivative according to an embodiment of the present invention as an active ingredient are also included in the scope of the present invention.

Embodiments of the present invention will be described in further detail hereinafter using examples. The present invention is of course not limited to the examples below, and it goes without saying that various aspects are possible for the details. Furthermore, the present invention is not limited to the embodiments described above, and various modifications are possible within the scope indicated in the claims. Embodiments obtained by appropriately combining the technical means disclosed by the embodiments are also included in the technical scope of the present invention. In addition, all of the documents described in the present specification are herein incorporated by reference.

[Examples]

[Synthesis of compounds]

<Synthesis Example 1. Synthesis of 1-((1H-1,2,4-triazole-1-yl)methyl)-5-(4-chlorophenoxy)-2,2-dimethyl-2,3-dihydro-1H-indene-1-ol (Compound No. I-7) (Synthesis Scheme 1)>

Synthesis of 5-fluoro-2,2-dimethyl-2,3-dihydro-1H-indene-1-one

9.61 g of 55% sodium hydride was weighed and washed with normal hexane. Then, 300 mL of N,N-dimethylformamide was added and cooled with stirring in an ice-water bath. To this, 15.02 g of 5-fluoroindanone was added in portions. Subsequently, 31.27 g of iodomethane was slowly added dropwise. After dripping, the mixture was stirred at room temperature for 2 hours. After the reaction, the reaction solution was poured into ice water and extracted with toluene. The organic layer was washed with water, a saturated salt solution, and dried with anhydrous sodium sulfate. The solvent was distilled off and the obtained crude was purified by silica gel column chromatography to yield 14.21 g of a target compound as a yellow solid. Yield: 80.0%.
$^1$H NMR (400 MHz, $CDCl_3$) $\delta$: 7.76 (dd, J = 8.1, 5.2 Hz, 1H), 7.10-7.06 (m, 2H), 2.99 (s, 2H), 1.24 (s, 6H).

Synthesis 1 of 5-(4-chlorophenoxy)-2,2-dimethyl-2,3-dihydro-1H-indene-1-one

8.91 g of 5-fluoro-2,2-dimethyl-2,3-dihydro-1H-indene-1-one synthesized in the previous section was weighed, 90 mL of N,N-dimethylformamide, 12.86 g of 4-chlorophenol, and 13.82 g of potassium carbonate were added and heated at 120°C for 6 hours. The reaction solution was allowed to cool and poured into ice water. The mixture was extracted with toluene, the organic layer was washed with 1N aqueous sodium hydroxide aqueous solution, water, and saturated salt solution, and dried over anhydrous sodium sulfate. The solvent was distilled off and the obtained crude was purified by silica gel column chromatography to yield 13.82 g of a target compound as a yellow liquid. Yield: 92.8%.
$^1$H NMR (400 MHz, $CDCl_3$) $\delta$: 7.73 (d, J = 8.4 Hz, 1H), 7.37 (d, J = 8.9 Hz, 2H), 7.03 (d, J = 8.8 Hz, 2H), 6.97 (dd, J = 8.4, 2.2 Hz, 1H), 6.88 (s, 1H), 2.92 (s, 2H), 1.23 (s, 6H).

Synthesis 2 of 5-(4-chlorophenoxy)-2,2-dimethyl-2,3-dihydro-1H-indene-1-one

239.4 mg of 5-bromo-2,2-dimethyl-2,3-dihydro-1H-indene-1-one, 194.0 mg of p-chlorophenol, 5 mL of N,N-dimethylformamide, 277.8 mg of potassium carbonate, 38.3 mg of copper iodide, and 139.8 mg of tris(2,4-pentanedionato) iron (III) were weighed, and heated and stirred at 135°C for 9 hours. The reaction solution was cooled to room temperature, water was added and extracted with toluene, the organic layer was washed with water and a saturated salt solution, and dried over anhydrous sodium sulfate. The solvent was distilled off, and 178.9 mg of a crude material was obtained. The resulting crude was purified by silica gel column chromatography to yield 104.8 mg of the title compound. Yield: 36.5%.

Synthesis of 1-((1H-1,2,4-triazole-1-yl)methyl)-5-(4-chlorophenoxy)-2,2-dimethyl-2,3-dihydro-1H-indene-1-ol (Compound No. I-7)

104.8 mg of 5-(4-chlorophenoxy)-2,2-dimethyl-2,3-dihydro-1H-indene-1-one synthesized in the previous section was weighed, and 1 mL of N-methylpyrrolidone and 66.8 mg of triazole sodium salt were added and heated to 80°C. To this, 76.1 mg of trimethylsulfoxonium bromide and 42.6 mg of sodium tert-butoxide were each divided into four portions and added every 30 minutes. The oil bath temperature was increased to 90°C after 70 minutes of the initial reagent addition, and increased to 100°C after 130 minutes of the initial reagent addition. Three hours after the initial reagent addition, the reaction solution was cooled to room temperature and water was added and extracted with toluene. The obtained organic layer was washed with water and a saturated salt solution, and dried with anhydrous sodium sulfate. The solvent was distilled off and the obtained crude was purified by silica gel column chromatography to give 61.1 mg of a target compound as a white solid. Yield: 45.3%.
$^1$H NMR (400 MHz, $CDCl_3$) $\delta$: 7.97 (s, 1H), 7.92 (s, 1H), 7.28 (dd, J = 9.0, 2.2 Hz, 2H), 6.90 (d, J = 9.0 Hz, 2H), 6.80 (s, 1H), 6.64 (d, J = 8.2 Hz, 1H), 6.33 (d, J = 8.2 Hz, 1H), 4.44 (d, J = 13.8 Hz, 1H), 4.25 (d, J = 13.8 Hz, 1H), 3.79 (s, 1H), 2.74 (s, 2H), 1.26 (s, 3H), 1.01 (s, 3H).

<Synthesis Example 2. Synthesis 2 of 1-((1H-1,2,4-triazole-1-yl)methyl)-5-(4-chlorophenoxy)-2,2-dimethyl-2,3-dihydro-1H-indene-1-ol (Compound No. I-7) (Synthesis Scheme 1')>

Synthesis of 1-((1H-1,2,4-triazole-1-yl)methyl)-5-bromo-2,2-dimethyl-2,3-dihydro-1H-indene-1-ol

654.7 mg of 5-bromo-2,2-dimethyl-2,3-dihydro-1H-indene-1-one was weighed, and 8.3 mL of N-methylpyrrolidone and 500.8 mg of triazole sodium salt were added and heated to 80°C. 142.8 mg of trimethylsulfoxonium bromide and 82.5 mg of sodium tert-butoxide were each divided into four portions and added to the mixture every 30 minutes. The oil bath temperature was increased to 90°C after 70 minutes of the initial reagent addition, and increased to 100°C after 130 minutes of the initial reagent addition. Three hours after the initial reagent addition, the reaction solution was cooled to room temperature and water was added and extracted with toluene. The obtained organic layer was washed with water and a saturated salt solution, and dried over anhydrous sodium sulfate. The solvent was distilled off and the obtained crude was purified by silica gel column chromatography to give 462.2 mg of a target compound as a brown liquid. Yield: 53%.
$^1$H NMR(400 MHz, $CDCl_3$) $\delta$: 7.98 (s, 1H), 7.87 (s, 1H), 7.34 (s, 1H), 7.14 (d, J = 8.0 Hz, 1H), 6.21 (d, J = 8.0 Hz, 1H), 4.42 (d, J = 14.0 Hz, 1H), 4.19 (d, J = 14.0 Hz, 1H), 3.94 (s, 1H), 2.80 (d, J = 16.0 Hz, 1H), 2.74 (d, J = 16.0 Hz, 1H), 1.27 (s, 3H), 0.99 (s, 3H).

Synthesis of 1-((1H-1,2,4-triazole-1-yl)methyl)-5-(4-chlorophenoxy)-2,2-dimethyl-2,3-dihydro-1H-indene-1-ol (Compound No. I-7)

365.2 mg of 1-((1H-1,2,4-triazole-1-yl)methyl)-5-bromo-2,2-dimethyl-2,3-dihydro-1H-indene-1-ol synthesized

in the previous section was dissolved in 5.7 mL of N,N-dimethylformamide, 365.1 mg of potassium carbonate, 218.9 mg of 4-chlorophenol, 43.4 mg of copper iodide, and 161.0 mg of tris(2,4-pentanedionato) iron (III) was added, and the mixture was stirred at 135°C for 9 hours. Water was added to the obtained reaction solution, and extraction with toluene was then carried out. The organic layer was washed with a saturated salt solution, and dried over anhydrous sodium sulfate. The solvent was distilled off and the resulting crude product was purified by silica gel column chromatography to give 51.7 mg of the title compound. Yield: 12.3%. $^1$H NMR (400 MHz, $CDCl_3$) 7.97 (s, 1H), 7.92 (s, 1H), 7.28 (dd, J = 9.0, 2.2 Hz, 2H), 6.90 (d, J = 9.0 Hz, 2H), 6.80 (s, 1H), 6.64 (d, J = 8.2 Hz, 1H), 6.33 (d, J = 8.2 Hz, 1H), 4.44 (d, J = 13.8 Hz, 1H), 4.25 (d, J = 13.8 Hz, 1H), 3.79 (s, 1H), 2.74 (s, 2H), 1.26 (s, 3H), 1.01 (s, 3H).

<Synthesis Example 3. Synthesis of 1-((1H-1,2,4-triazole-1-yl)methyl)-5-(4-chlorophenoxy)-2,3-dihydro-1H-indene-1-ol (Compound No. I-27) (Synthesis Scheme 2)

Synthesis of 5-(4-chlorophenoxy)-2,3-dihydro-1 H-indene-1-one

301 mg of 5-fluoroindanone and 515 mg of 4-chlorophenol were dissolved in 3.6 mL of N,N-dimethylformamide, and 553 mg of potassium carbonate was added. After heating and stirring at 120°C for 4 hours, the reaction solution was cooled to room temperature and water was added. The mixture was extracted with toluene, and the obtained organic layer was washed with water and passed through a diatomaceous earth column. The solvent was distilled off and the obtained crude was purified by silica gel column chromatography to give 314 mg of a target compound as a brown oily substance. Yield: 60.5%.
$^1$H NMR (400 MHz, $CDCl_3$) δ: 7.73 (d, J = 8.4 Hz, 1H), 7.37 (d, J = 9.0 Hz, 2H), 7.03 (d, J = 9.0 Hz, 2H), 6.97 (dd, J = 8.4, 2.2 Hz, 1H), 6.93 (s, 1H), 3.09-3.06 (m, 2H), 2.71-2.68 (m, 2H).

Synthesis of 5-(4-chlorophenoxy)-1-methylene-2,3-dihydro-1H-indene

1.294 g of 5-(4-chlorophenoxy)-2,3-dihydro-1H-indene-1-one, 3.572 g of methyl triphenylphosphonium bromide, and 20 mL of dehydrated tetrahydrofuran were weighed, and while stirring at room temperature, a liquid mixture of 1.122 g of potassium tert-butoxide and 10 mL of dehydrated tetrahydrofuran was added dropwise for 2 hours. The reaction solution was concentrated to one-third extent by stirring for 1 hour after dripping. Since precipitation occurred when hexane was added thereto, the precipitation was removed by celite filtration, and the filtrate was washed with water and saturated salt solution, and the organic layer was passed through a diatomaceous earth column. The solvent was distilled off and the obtained crude was purified by silica gel column chromatography to give 1.025 g of a target compound as a colorless oily substance. Yield: 79.9%.
$^1$H NMR (400 MHz, $CDCl_3$) δ:7.45 (dd, J = 7.0, 2.2 Hz, 1H), 7.30-7.24 (m, 2H), 6.99-6.90 (m, 2H), 6.86-6.83 (m, 2H), 5.36 (t, J = 2.4 Hz, 1H), 5.99 (t, J = 2.1 Hz, 1H), 2.96-2.90 (m, 2H), 2.86-2.80 (m, 2H).

Synthesis of 5-(4-chlorophenoxy)-1-(hydroxymethyl)-2,3-dihydro-1H-indene-1-ol

1.133 g of 50% N-methylmorpholine N-oxide aqueous solution was weighed and 1.0 mL of water was added. A small amount of osmium tetraoxide was added, and 1.025 g of 5-(4-chlorophenoxy)-1-methylene-2,3-dihydro-1H-indene as synthesized in the previous section, and 0.8 mL of acetone were added. The reaction solution was stirred vigorously for 9 hours at room temperature, and a sodium thiosulfate aqueous solution was added to the reaction solution. Ethyl acetate was added and the insoluble matter was removed by celite filtration. The filtrate was extracted with ethyl acetate and washed with water and saturated salt solution and the organic layer was passed through a diatomaceous earth column. The solvent was distilled off, and the obtained crude was purified by silica gel column chromatography to obtain 0.850 g of a target compound as a gray solid. Yield: 73.2%.
$^1$H NMR (400 MHz, DMSO-$d_6$) δ: 7.42 (d, J = 9.0 Hz, 2H), 7.31-7.29 (m, 1H), 6.99 (d, J = 9.0 Hz, 2H), 6.87-6.84 (m, 2H), 4.99 (s, 1H), 4.68 (t, J = 5.8 Hz, 1H), 3.45 (d, J = 5.8 Hz, 2H), 2.89-2.81 (m, 1H), 2.75-2.67 (m, 1H), 2.35-2.28 (m, 1H), 1. 93-1.83 (m, 1 H).

Synthesis of (5-(4-chlorophenoxy)-1-hydroxy-2,3-dihydro-1H-indene-1-yl) methyl 4-methylbenzensulfonate

0.850 g of 5-(4-chlorophenoxy)-1-(hydroxymethyl)-2,3-dihydro-1H-indene-1-ol synthesized in the previous section, and 2.9 mL of chloroform were weighed, and 0.447 g of pyridine and 0.838 g of p-toluenesulfonyl chloride were added under ice-water cooling and stirred for 5 hours in an ice-water bath. After the reaction, saturated aqueous sodium bicarbonate solution was added to the reaction solution and extracted with ethyl acetate, and the organic layer was passed through a diatomaceous earth column. The solvent was distilled off, and the obtained crude was purified by silica gel column chromatography to give 0.686 g of a target compound as a colorless oily substance. Yield: 52.7%.

$^1H$ NMR (400 MHz, DMSO-$d_6$) δ: 7.74 (d, J = 8.3 Hz, 2H), 7.46-7.42 (m, 4H), 7.20 (d, J = 8.0 Hz, 1H), 7.02-6.99 (m, 2H), 6.84-6.81 (m, 2H), 5.56 (br, 1H), 4.06-3.97 (m, 2H), 2.87-2.80 (m, 1H), 2.69-2.61 (m, 1H), 2.42 (s, 3H), 2.18-2.12 (m, 1H), 1.98-1.90 (m, 1H).

Synthesis of 1-((1H-1,2,4-triazole-1-yl)methyl)-5-(4-chlorophenoxy)-2,3-dihydro-1H-indene-1-ol (Compound No. I-27)

0.686 g of (5-(4-chlorophenoxy)-1-hydroxy-2,3-dihydro-1H-indene-1-yl) methyl 4-methylbenzenesulfonate, 1.5 mL of N-methylpyrrolidone, and 0.280 g of triazole sodium salt were added and stirred at 60°C for 1 hour. After the reaction, a saturated ammonium chloride aqueous solution was added to the reaction solution and extracted with ethyl acetate, and the organic layer was passed through a diatomaceous earth column. The solvent was distilled off, and the obtained crude was purified by silica gel column chromatography to give 0.081 mg of a target compound as a yellow oily substance. Yield: 15.4%. $^1H$ NMR (400 MHz, DMSO-$d_6$) δ:8.04 (s, 1H), 8.01 (s, 1H), 7.29 (dd, J = 6.8, 2.2 Hz, 2H), 6.92 (dd, J = 6.7, 2.2 Hz, 2H), 6.98-6.80 (m, 3H), 4.38-4.35 (m, 2H), 3.72 (s, 1H), 3.01-2.96 (m, 1H), 2.85-2.77 (m, 1H), 2.38-2.32 (m, 1H), 2.17-2.09 (m, 1H).

<Synthesis Example 4. Synthesis of 5-(4-chlorophenoxy)-2-isopropyl-2,3-dihydro-1H-indene-1-one (Synthesis Scheme 2)>

Synthesis of methyl 5-(4-chlorophenoxy)-1-oxo-2,3-dihydro-1H-indene-2-carboxylate

134 mg of 55% sodium hydride was weighed and washed with hexane. To this, 1.0 mL of dimethyl carbonate and 10 μL of dehydrated methanol were added and heated to 80°C. After heating, a liquid mixture of 313.7 mg of 5-(4-chlorophenoxy)-2,3-dihydro-1H-indene-1-one synthesized in the previous section and 3.0 mL of dimethyl carbonate were added. After heating under reflux at 100°C for 1.5 hours, the reaction solution was poured into ice water after cooling. A saturated ammonium chloride aqueous solution was added and extracted with toluene, and the obtained organic layer was washed with water and passed through a diatomaceous earth column. The solvent was distilled off, and the obtained crude was purified by silica gel column chromatography to give 228 mg of a target compound as a brown oily substance. Yield: 59.4%. $^1H$ NMR (400 MHz, $CDCl_3$) δ: 7.74 (d, J = 8.5 Hz, 1H), 7.40-7.37 (m, 2H), 7.05-7.03 (m, 2H), 7.01-6.98 (m, 1H), 6.93 (s, 1H), 3.80 (s, 3H), 3.74 (dd, J = 8.3, 4.0 Hz, 1H), 3.49 (dd, J = 17.4, 3.8 Hz, 1H), 3.28 (dd, J = 17.4, 8.2 Hz, 1H).

Synthesis of methyl 5-(4-chlorophenoxy)-2-isopropyl-1-oxo-2,3-dihydro-1H-indene-2-carboxylate

35.6 mg of 55% sodium hydride was weighed and washed with hexane. To this, 1.0 mL of N,N-dimethylformamide was added and cooled in an ice-water bath, and a liquid mixture of 228.3 mg of methyl 5-(4-chlorophenoxy)-1-oxo-2,3-dihydro-1H-indene-2-carboxylate and 1.2 mL of N,N-dimethylformamide were added. 134.8 mg of isopropyl iodide was then added and heated at 80°C for 1.5 hours. The reaction solution was cooled to room temperature and a saturated ammonium chloride aqueous solution was added. The resultant was extracted with ethyl acetate, and the obtained organic layer was washed with water and a saturated salt solution, and dried with anhydrous sodium sulfate. The solvent was distilled off, and the obtained crude was purified by silica gel column chromatography to give 93.7 mg of a target compound as a yellow oily substance. Yield: 36.2%.
$^1H$ NMR (400 MHz, $CDCl_3$) δ: 7.70 (d, J = 8.4 Hz, 1H), 7.39 (d, J = 8.9 Hz, 2H), 7.05 (d, J = 8.9 Hz, 2H), 6.97-6.93 (m, 2H), 3.72 (s, 3H), 3.64 (d, J = 17.7 Hz, 1H), 2.99 (d, J = 17.7 Hz, 1H), 2.92-2.85 (m, 1H), 0.96 (d, J = 6.8 Hz, 3H), 0.74 (d, J = 6.8 Hz, 3H).

Synthesis of 5-(4-chlorophenoxy)-2-isopropyl-2,3-dihydro-1H-indene-1-one

1.1 mL of tetrahydrofuran and 1.1 mL of a 30 wt.% sodium hydroxide aqueous solution were added to 90.1 mg of ethyl 5-(4-chlorophenoxy)-2-isopropyl-1-oxo-2,3-dihydro-1H-indene-2-carboxylate synthesized in the previous section, and the mixture was heated under for 4.5 hours. After cooling the reaction solution, it was neutralized with dilute hydrochloric acid and extracted with toluene. The obtained organic layer was washed with water and passed through a diatomaceous earth column. The solvent was distilled off, and the obtained crude was purified by silica gel column chromatography to give 50.0 mg of a target compound as a white solid. Yield: 66.2%.
$^1H$ NMR (400 MHz, $CDCl_3$) δ: 7.70 (d, J = 8.4 Hz, 1H), 7.37 (d, J = 8.9 Hz, 2H), 7.03 (d, J = 9.0 Hz, 2H), 6.96-6.92 (m, 2H), 3.06 (dd, J = 17.6, 8.1 Hz, 1H), 2.85 (dd, J = 17.6, 4.0 Hz, 1H), 2.69-2.65 (m, 1H), 2.44~2.36 (m, 1H), 1.04 (d, J = 6.9 Hz, 3H), 0.79 (d, J = 6.8 Hz, 3H).

<Synthesis Example 5. Synthesis of 1-((1H-1,2,4-triazole-1-yl)methyl)-5-(4-chlorophenoxy)-2-methyl-2,3-dihydro-1H-indene-1-ol (Compound No. I-32 and I-33) (Synthesis Scheme 2')>

Synthesis of methyl 5-bromo-1-oxo-2,3-dihydro-1H-indene-2-carboxylate

4.813 g of 55% sodium hydride, 250 mL of dimethyl carbonate, and 1 mL of dehydrated methanol were weighed, and a mixed solution of 21.11 g of 5-bromo-1-indanone and 150 mL of dimethyl carbonate was added dropwise while heating at 50°C. After dripping, the mixture was stirred for 30 minutes, and the reaction solution was poured into ice water. 200 mL of saturated ammonium chloride aqueous solution was added, the mixture was extracted with ethyl acetate, and the organic layer was washed with water and a saturated salt solution, and dried over anhydrous sodium sulfate. The solvent was distilled off, and the obtained crude was purified by silica gel column chromatography to give 19.17 g of a target compound as a light yellow solid. Yield: 71.2%.
$^1$H NMR (400 MHz, $CDCl_3$) δ: 7.70 (d, J = 0.9 Hz, 1H), 7.64 (d, J = 8.1 Hz, 1H), 7.56-7.53 (m, 1H), 3.80 (s, 3H), 3.74 (dd, J = 8.3, 4.0 Hz, 1H), 3.56 (dd, J = 17.4, 4.0 Hz, 1H), 3.36 (dd, J = 17.4, 8.3 Hz, 1H).

Synthesis of methyl 5-bromo-2-methyl-1-oxo-2,3-dihydro-1H-indene-2-carboxylate

18.90 g of methyl 5-bromo-1-oxo-2,3-dihydro-1H-indene-2-carboxylate synthesized in the previous section, 19.44 g of potassium carbonate, and 70 mL of N,N-dimethylformamide were weighed, and stirred at room temperature, and 19.94 g of methyl iodide was added dropwise. After dripping, the mixture was stirred for 1 hour, water was added, and the mixture was extracted with ethyl acetate. The obtained organic layer was washed with water and a saturated salt solution, and dried with anhydrous sodium sulfate. The solvent was distilled off, and the obtained crude was purified by silica gel column chromatography to give 18.96 g of a target compound as a yellow solid. Yield: 95.4%.
$^1$H NMR (400 MHz, $CDCl_3$) δ:7.66-7.64 (m, 2H), 7.56 (d, J = 8.2 Hz, 1H), 3.70 (d, J = 17.3 Hz, 1H), 3.69 (s, 3H), 2.98 (d, J = 17.4 Hz, 1H), 1.52 (s, 3H).

Synthesis of 5-bromo-2-methyl-2,3-dihydro-1H-indene-1-one

18.41 g of methyl 5-bromo-2-methyl-1-oxo-2,3-dihydro-1H-indene-2-carboxylate and 260 mL of tetrahydrofuran were weighed, and 260 g of 30 wt.% sodium hydroxide aqueous solution were added, and the mixture was heated under reflux for 2 hours while strongly stirring. After cooling the reaction solution to room temperature, it was neutralized with dilute hydrochloric acid and extracted with ethyl acetate. The obtained organic layer was washed with water and a saturated salt solution, and dried with anhydrous sodium sulfate. The solvent was distilled off, and the obtained crude was purified by silica gel column chromatography to give 4.810 g of a target compound as a yellow solid. Yield: 32.9%.
$^1$H NMR (400 MHz, $CDCl_3$) δ: 7.63 (s, 1H), 7.61 (d, J = 8.2 Hz, 1H), 7.51 (d, J = 8.1 Hz, 1H), 3.42-3.35 (m, 1H), 2.74-2.70 (m, 2H), 1.31 (d, J = 7.3 Hz, 3H).

Synthesis of 5-bromo-2-methyl-1-methylene-2,3-dihydro-1H-indene

4.810 g of 5-bromo-2-methyl-2,3-dihydro-1H-indene-1-one synthesized in the previous section, 15.27 g of methyl triphenylphosphonium bromide, and 85 mL of dehydrated tetrahydrofuran were weighed, and while stirring at room temperature, a liquid mixture of 4.783 g of potassium tert-butoxide and 43 mL of dehydrated tetrahydrofuran was added dropwise for 0.5 hours. The reaction solution was concentrated to one-fourth extent by stirring for 1 hour after dripping. Since precipitation occurred when hexane was added thereto, the precipitation was removed by celite filtration, and the filtrate was washed with water and saturated salt solution, and the organic layer was passed through a diatomaceous earth column. The solvent was distilled off, and the obtained crude was purified by silica gel column chromatography to give 4.528 g of a target compound as a colorless oily substance. Yield: 94.9%
$^1$H NMR (400 MHz, $CDCl_3$) δ: 7.37 (s, 1H), 7.34-7.31 (m, 2H), 5.45 (d, J = 2.6 Hz, 1H), 5.00 (d, J = 2.2 Hz, 1H), 3.16 (dd, J = 16.2, 8.5 Hz, 1H), 3.05-3.02 (m, 1H), 2.55 (dd, J = 16.2, 5.1 Hz, 1H), 1.24 (d, J = 7.0 Hz, 3H)..

Synthesis of 5-bromo-1-(hydroxymethyl)-2-methyl-2,3-dihydro-1H-indene-1-ol

5.715 g of 50% N-methylmorpholine N-oxide aqueous solution was weighed and 5.2 mL of water was added. 20.2 mg of osmium tetraoxide was added, and 4.528 g of 5-bromo-2-methyl-1-methylene-2,3-dihydro-1H-indene synthesized in the previous section and 4.1 mL of acetone were added. The reaction solution was stirred vigorously for 6 hours at room temperature, and a sodium thiosulfate aqueous solution was added to the reaction solution. Ethyl acetate was added and the insoluble matter was removed by celite filtration. The filtrate was extracted with ethyl acetate and washed with water and saturated salt solution and the organic layer was passed through a diatomaceous earth column.

The solvent was distilled off, and the obtained crude was purified by silica gel column chromatography to give 4.768 g of a target compound as a brown oily substance. Yield: 91.4%.

Diastereomer 1

$^1$H NMR (400 MHz, DMSO-$d_6$) δ:7.37-7.22 (m, 3H), 5.02 (s, 1H), 4.39 (t, J = 5.3 Hz, 1H), 3.46-3.41 (m, 2H), 2.94-2.85 (m, 1H), 2.56-2.46 (m, 1H), 2.32-2.29 (m, 1H), 1.12 (d, J = 7.0 Hz, 3H).

Diastereomer 2

$^1$H NMR (400 MHz, DMSO-$d_6$) δ:7.37-7.22 (m, 3H), 4.68-4.65 (m, 2H), 3.57 (dd, J = 11.0, 5.7 Hz, 1H), 3.46-3.41 (m, 1H), 2.94-2.85 (m, 1H), 2.56-2.46 (m, 1H), 2.44-2.38 (m, 1H), 0.96 (d, J = 6.9 Hz, 3H).

Synthesis of (5-bromo-1-hydroxy-2-methyl-2,3-dihydro-1H-indene-1-yl) methyl 4-methylbenzenesulfonate

4.768 g of 5-bromo-1-(hydroxymethyl)-2-methyl-2,3-dihydro-1H-indene-1-ol synthesized in the previous section and 19 mL of chloroform were weighed, and 2.937 g of pyridine and 5.300 g of p-toluenesulfonyl chloride were added under ice-water cooling and stirred for 5.5 hours in an ice-water bath. After the reaction, water was added to the reaction solution, the mixture was extracted with ethyl acetate, and the organic layer was passed through a diatomaceous earth column. The solvent was distilled off, and the obtained crude was purified by silica gel column chromatography to give 7.549 g of a target compound as a green transparent oily substance. Yield: 99.0%.

Diastereomer 1

$^1$H NMR (400 MHz, $CDCl_3$) δ: 7.61 (d, J = 8.3 Hz, 2H), 7.35-7.25 (m, 4H), 7.08 (d, J = 8.1 Hz, 1H), 4.14 (d, J = 10.0 Hz, 1H), 4.05 (d, J = 10.0 Hz, 1H), 3.05-2.93 (m, 1H), 2.50-2.41 (m, 5H), 1.15 (d, J = 6.7 Hz, 3H).

Diastereomer 2

$^1$H NMR (400 MHz, $CDCl_3$) δ: 7.76 (d, J = 8.3 Hz, 2H), 7.35-7.25 (m, 4H), 7.12 (d, J = 8.1 Hz, 1H), 4.15-4.10 (m, 2H), 3.05-2.93 (m, 1H), 2.61 (dd, J = 16.2, 6.1 Hz, 1H), 2.50-2.41 (m, 4H), 1.06 (d, J = 7.0 Hz, 3H).

Synthesis of 1-((1H-1,2,4-triazole-1-yl)methyl)-5-bromo-2-methyl-2,3-dihydro-1H-indene-1-ol

7.549 g of (5-bromo-1-hydroxy-2-methyl-2,3-dihydro-1H-indene-1-yl) methyl 4-methylbenzenesulfonate, 18 mL of N-methylpyrrolidone, and 3.346 g of triazole sodium salt were added and stirred at 60°C for 1.5 hours. After the reaction, a saturated ammonium chloride aqueous solution was added to the reaction solution and extracted with ethyl acetate, and the organic layer was passed through a diatomaceous earth column. The solvent was distilled off, and the obtained crude was purified by silica gel column chromatography to obtain 3.539 mg of a target compound as a white solid. Yield: 62.6%.

Diastereomer 1

$^1$H NMR (400 MHz, $CDCl_3$) δ: 8.01 (s, 1H), 7.81 (s, 1H), 7.38-7.35 (m, 1H), 7.13 (d, J = 6.8 Hz, 1H), 6.14 (d, J = 8.0 Hz, 1H), 4.40 (d, J = 13.6 Hz, 1H), 4.27 (s, 1H), 4.08 (d, J = 13.8 Hz, 1H), 3.10-3.03 (m, 1H), 2.64-2.51 (m, 2H), 1.29 (d, J = 6.9 Hz, 3H).

Diastereomer 2

$^1$H NMR (400 MHz, $CDCl_3$) δ: 8.06 (s, 1H), 7.98 (s, 1H), 7.38-7.35 (m, 1H), 7.32 (d, J = 8.0 Hz, 1H), 6.81 (d, J = 8.1 Hz, 1H), 4.40 (d, J = 13.6 Hz, 1H), 4.34 (d, J = 14.0 Hz, 1H), 3.49 (s, 1H), 3.10-3.03 (m, 1H), 2.64-2.51 (m, 1H), 2.47-2.44 (m, 1H), 0.91 (d, J = 7.0 Hz, 3H).

Synthesis of 1-((1H-1,2,4-triazole-1-yl)methyl)-5-(4-chlorophenoxy)-2-methyl-2,3-dihydro-1H-indene-1-ol (Compound No. I-32, I-33)

306.2 mg of 1-((1H-1,2,4-triazole-1-yl)methyl)-5-bromo-2-methyl-2,3-dihydro-1H-indene-1-ol synthesized in the previous section was dissolved in 1.5 mL of N,N-dimethylformamide, and 653.0 mg of cesium carbonate, 372 mg of 4-chlorophenol, and 20 mg of copper iodide were weighed, and the mixture was stirred at 195°C for 2 hours using a microwave synthesizer. 1 N sodium hydroxide aqueous solution was added to the obtained solution, and extracted with ethyl acetate. The organic layer was washed with water and a saturated salt solution, and passed through a diatomaceous earth column. The solvent was distilled off and the resulting crude product was purified by silica gel column chromatography to give 121.4 mg of the title compound. Yield: 34.9%. Subsequently, the diastereomers were fractionated on a preparative column.

Diastereomer 1: (Compound No. I-32)

$^1$H NMR (600 MHz, $CDCl_3$) $\delta$: 8.01 (s, 1H), 7.85 (s, 1H), 7.27 (dd, J = 6.5, 2.4 Hz, 2H), 6.89 (dd, J = 6.9, 2.1 Hz, 2H), 6.80 (d, J = 2.1 Hz, 1H), 6.64 (dd, J = 8.3, 2.8 Hz, 1H), 6.25 (d, J = 8.3 Hz, 1H), 4.42 (d, J = 13.8 Hz, 1H), 4.13-4.11 (m, 2H), 3.08-3.04 (m, 1H), 2.64-2.61 (m, 1H), 2.51 (dd, J = 15.8, 10.3 Hz, 1H), 1.29 (d, J = 6.9 Hz, 3H).

Diastereomer 2: (Compound No. I-33)

$^1$H NMR (600 MHz, $CDCl_3$) $\delta$: 8.10 (s, 1H), 7.99 (s, 1H), 7.29 (dd, J = 6.2, 2.1 Hz, 2H), 6.95-6.92 (m, 3H), 6.83-6.82 (m, 2H), 4.47 (d, J = 14.5 Hz, 1H), 4.37 (d, J = 13.8 Hz, 1H), 3.29 (s, 1H), 3.03-2.99 (m, 1H), 2.61-2.58 (m, 1H), 2.47-2.44 (m, 1H), 0.90 (d, J = 6.9 Hz, 3H).

<Synthesis Example 6. Synthesis of 1'-((1H-1,2,4-triazole-1-yl)methyl)-5'-(4-chlorophenoxy)-1',3'-dihydrospiro[cyclopropane-1,2'-indene]-1'-ol (Compound No. I-17) (Synthesis Scheme 3)>

Synthesis of 5-fluoro-2-methylene-2,3-dihydro-1H-indene-1-one

1.350 g of paraformaldehyde and 3.317 g of N-methylanilinium Trifluoroacetate were weighed, and a liquid mixture of 1.503 g of 5-fluoroindanone and 10 mL of tetrahydrofuran was added. The mixture was heated under reflux for 3 hours, cooled to room temperature, 20 mL of diethyl ether was added, and the mixture was stirred and decanted. The organic layer was washed with a two-fold dilution of saturated aqueous sodium bicarbonate solution, further washed with water, and passed through a diatomaceous earth column. The solvent was distilled off at 30°C to obtain 1.313 g of a target compound as a dark green solid. Yield: 80.9%.
$^1$H NMR (400 MHz, $CDCl_3$) $\delta$:7.89 (dd, J = 8.4, 5.4 Hz, 1H), 7.18-7.10 (m, 2H), 6.37-6.36 (m, 1H), 5.66-5.65 (m, 1H), 3.76 (s, 2H).

Synthesis of 5'-fluorospiro [cyclopropane-1,2'-indene]-1'(3'H) -one

0.364 g of 55% sodium hydride was weighed and washed with hexane. 10 mL of dimethyl sulfoxide was added and 1.839 g of trimethylsulfoxonium iodide was added in two portions. The mixture was stirred at room temperature for 15 minutes, and a mixed solution of 1.232 g of 5-fluoro-2-methylene-2,3-dihydro-1H-indene-1-one and 10 mL of dimethyl sulfoxide was added and washed with 2.8 mL of dimethyl sulfoxide. The mixture was stirred at room temperature for 1 hour and the reaction solution was poured into ice water and the saturated ammonium chloride aqueous solution was added. The mixture was extracted with toluene, and the obtained organic layer was washed with water and passed through a diatomaceous earth column. The solvent was distilled off, and the obtained crude was purified by silica gel column chromatography to give 0.523 g of a target compound as a white crystal. Yield: 39.0%.
$^1$H NMR (400 MHz, $CDCl_3$) $\delta$: 7.78 (dd, J = 8.5, 5.4 Hz, 1H), 7.16 (d, J = 8.6 Hz, 1H), 7.10 (td, J = 8.9, 2.3 Hz, 1H), 3.22 (s, 2H), 1.47-1.44 (m, 2H), 1.17-1.15 (m, 2H).

Synthesis of 5'-(4-chlorophenoxy)spiro[cyclopropane-1,2'-inden]-1' (3'H)-one

353 mg of 5'-fluorospiro [cyclopropane-1,2'-indene]-1'(3'H) -one synthesized in the previous section was weighed, 3.6 mL of N,N-dimethylformamide, 514 mg of 4-chlorophenol, and 554 mg of potassium carbonate were heated and stirred at 120°C for 6 hours. After the reaction, the reaction solution was cooled to room temperature and water was added. The mixture was extracted with toluene, and the obtained organic layer was washed with 1M NaOH aqueous solution and water, and passed through a diatomaceous earth column. The solvent was distilled off, and the obtained crude was purified by silica gel column chromatography to give 499 mg of a target compound as a colorless oily substance. Yield: 87.4%.
$^1$H NMR (400 MHz, $CDCl_3$) $\delta$: 7.75 (d, J = 8.4 Hz, 1H), 7.39-7.35 (m, 2H), 7.05-6.99 (m, 3H), 6.97 (s, 1H), 3.15 (s, 2H), 1.45-1.42 (m, 2H), 1.14-1.11 (m, 2H).

Synthesis of 1'-((1H-1,2,4-triazole-1-yl)methyl)-5'-(4-chlorophenoxy)-1',3'-dihydrospiro[cyclopropane-1,2'-indene]-1'-ol (Compound No. I-17)

497 mg of 5'-(4-chlorophenoxy)spiro[cyclopropane-1,2'-inden]-1' (3'H)-one synthesized in the previous section was dissolved in 5.2 mL of N-methylpyrrolidone, and 319 mg of triazole sodium salt was added and heated to 80°C. 363 mg of trimethylsulfoxonium bromide and 202 mg of sodium tert-butoxide were each divided into four portions and added to the mixture every 30 minutes. The oil bath temperature was increased to 90°C after 70 minutes of the initial reagent addition, and increased to 100°C after 130 minutes of the initial reagent addition. Three hours after the initial reagent addition, the reaction solution was cooled to room temperature and water was added and extracted with toluene. The obtained organic layer was washed with water and passed through a diatomaceous earth column. The solvent was distilled off, and the obtained crude was purified by silica gel column chromatography to give 59.1 mg of a target compound as a white solid. Yield: 9.2%.
$^1$H NMR (400 MHz, $CDCl_3$) 7.95 (s, 1H), 7.91 (s, 1H), 7.29 (d, J = 8.7 Hz, 2H), 6.92 (d, J = 8.8 Hz, 2H), 6.79 (s, 1H), 6.73 (d, J = 8.3 Hz, 1H), 6.50 (d, J = 8.3 Hz, 1H), 4.32 (d, J = 14.0 Hz, 1H), 4.28 (d, J = 14.2 Hz, 1H), 3.39 (s, 1H), 3.12 (d, J = 16.3 Hz, 1H), 2.55 (d, J = 16.4 Hz, 1H), 1.11-1.06 (m, 1H), 0.82-0.77 (m, 1H), 0.73-0.67 (m, 1H), 0.43-0.38 (m, 1H).

<Synthesis Example 7. Synthesis of 1-((1H-1,2,4-triazole-1-yl)methyl)-2,2-dimethyl-5-((5-(trifluoromethyl)pyridin-2-yl) oxy)-2,3-dihydro-1H-indene-1-ol (Compound No. I-18) (Synthesis Scheme 4)>

Synthesis of 5-(benzyloxy)-2,2-dimethyl-2,3-dihydro-1H-indene-1-one

1.782 g of 5-fluoro-2,2-dimethyl-2,3-dihydro-1H-indene-1-one, 1.191 g of benzyl alcohol, and 30 mL of N,N-dimethylformamide were weighed, and 1.235 g of potassium tert-butoxide was added under ice-bath cooling. The mixture was stirred at room temperature for 1 hour and water was added. The mixture was extracted with toluene, and the organic layer was washed once with a saturated salt solution and passed through a diatomaceous earth column. The solvent was distilled off, and the obtained crude was purified by silica gel column chromatography to give 2.450 g of a target compound. Yield: 92.0%.
$^1$H NMR (400 MHz, $CDCl_3$) $\delta$: 7.70 (d, J = 8.5 Hz, 1H), 7.45-7.36 (m, 5H), 6.99 (dd, J = 8.5, 2.3 Hz, 1H), 6.94 (s, 1H), 5.14 (s, 2H), 2.94 (s, 2H), 1.22 (s, 6H).

Synthesis of 1-((1H-1,2,4-triazole-1-yl)methyl)-5-(benzyloxy)-2,2-dimethyl-2,3-dihydro-1H-indene-1-ol

0.483 g of 55% sodium hydride was weighed and washed with hexane. Then, 14.4 mL of dehydrated dimethyl sulfoxide and 2.441 g of trimethylsulfonium iodide were added and stirred at 25°C for 30 minutes. A liquid mixture of 2.450 g of 5-(benzyloxy)-2,2-dimethyl-2,3-dihydro-1H-indene-1-one synthesized in the previous section and 2.0 mL of dehydrated dimethyl sulfoxide was added and washed with 2.0 mL of dehydrated dimethyl sulfoxide. The mixture was stirred at 25°C for 4 hours, then 1.278 g of 1,2,4-triazole and 2.809 g of diazabicycloundecene were added and stirred at 80°C for 3 hours. The reaction solution was cooled to room temperature and a saturated ammonium chloride aqueous solution was added under ice-cold conditions. The mixture was extracted with ethyl acetate, and the organic layer was washed with a saturated salt solution and passed through a diatomaceous earth column. The solvent was distilled off, and the obtained crude was purified by silica gel column chromatography to obtain 0.746 g of a target compound as a white solid. Yield: 23.2%.
$^1$H NMR (400 MHz, $CDCl_3$) $\delta$: 8.00 (s, 1H), 7.54 (s, 1H), 7.46-7.35 (m, 5H), 7.23 (d, J = 8.3 Hz, 1H), 6.94-6.90 (m, 2H), 5.09 (s, 2H), 4.37 (dd, J = 12.2, 9.0 Hz, 1H), 4.29 (dd, J = 12.2, 5.1 Hz, 1H), 3.67-3.64 (m, 1H), 2.83 (d, J = 16.1 Hz, 1H), 2.71 (d, J = 16.1 Hz, 1H), 1.29 (s, 3H), 0.75 (s, 3H).

1-((1H-1,2,4-triazole-1-yl)methyl)-2,2-dimethyl-2,3-dihydro-1H-indene-1,5-diol

488.8 mg of 1-((1H-1,2,4-triazole-1-yl)methyl)-5-(benzyloxy)-2,2-dimethyl-2,3-dihydro-1H-indene-1-ol synthesized in the previous section was suspended in 2.8 mL of ethanol, 129.8 mg of 10% palladium carbon (about 55% water wet) was added, and hydrogen was introduced by a balloon. The mixture was stirred at room temperature for 8 hours and the insoluble matter was removed by celite filtration. The filtrate was concentrated, and the obtained crude was purified by silica gel column chromatography to obtain 331.9 mg of a target compound as a white solid. Yield: 91.5%.
$^1$H NMR (400 MHz, DMSO-$d_6$) $\delta$: 9.43 (s, 1H), 8.31 (s, 1H), 7.99 (s, 1H), 7.17 (d, J = 8.1 Hz, 1H), 6.64 (m, 2H), 4.87 (t, J = 5.0 Hz, 1H), 4.30 (dd, J = 11.0, 4.8 Hz, 1H), 3.81 (dd, J = 11.0, 5.2 Hz, 1H), 2.75 (d, J = 15.6 Hz, 1H), 2.69 (d, J = 15.6 Hz, 1H), 1.19 (s, 3H), 0.38 (s, 3H).

Synthesis of 1-((1H-1,2,4-triazole-1-yl)methyl)-2,2-dimethyl-5-((5-(trifluoromethyl)pyridin-2-yl) oxy)-2,3-dihydro-1H-indene-1-ol (Compound No. 1-18)

130.1 mg of 1-((1H-1,2,4-triazole-1-yl)methyl)-2,2-dimethyl-2,3-dihydro-1H-indene-1,5-diol synthesized in the previous section, 124.2 mg of 2-fluoro-5-trifluoromethylpyridine, 249.9 mg of cesium carbonate, and 1.0 mL of dehydrated N,N-dimethylformamide were weighed and heated and stirred at 60°C for 4 hours. After the reaction, the reaction solution was cooled to room temperature, water was added, the mixture was extracted with ethyl acetate, and the organic layer was passed through a diatomaceous earth column. The solvent was distilled off, and the obtained crude was purified by silica gel column chromatography to give 124.1 mg of a target compound as a colorless oily substance. Yield: 61.1%.
$^1$H NMR (400 MHz, $CDCl_3$) δ: 8.47 (s, 1H), 8.04 (s, 1H), 7.95 (d, J = 8.6 Hz, 1H), 7.66 (s, 1H), 7.40 (d, J = 8.3 Hz, 1H), 7.14-7.07 (m, 3H), 4.47 (m, 1H), 4.28 (d, J = 12.1 Hz, 1H), 3.48 (s, 1H), 2.89 (d, J = 16.2 Hz, 1H), 2.80 (d, J = 16.2 Hz, 1H), 1.33 (s, 3H), 0.75 (s, 3H).

<Synthesis Example 8. Synthesis of other compounds>

Compounds corresponding to compound Nos I-1 to I-6, I-8 to I-16, and I-19 to I-38 were synthesized by appropriately changing the compound used and conditions in the synthesis examples 1 to 7 described above.

[Synthesis of salt]

<Synthesis Example 9. Synthesis of 1-((1H-1,2,4-triazole-1-yl)methyl)-5-(4-chlorophenoxy)-2,2-dimethyl-2,3-dihydro-1H-indene-1-ol hydrochloride (Compound No. I-S1)>

150 mg of 1-((1H-1,2,4-triazole-1-yl)methyl)-5-(4-chlorophenoxy)-2,2-dimethyl-2,3-dihydro-1H-indene-1-ol was dissolved in a mixed solvent of 2 mL of dioxane and 3 mL of chloroform, 0.2 mL of a 4M hydrogen chloride dioxane solution was added, and the mixture was stirred at room temperature for 2 hours. The precipitated solid was filtered off and washed with dioxane to yield 93 mg of target compound as a white solid. Yield: 56%.
$^1$H NMR (400 MHz, DMSO-$d_6$) δ:8.39 (s, 1H), 7.94 (s, 1H), 7.42 (d, J = 8.8 Hz, 2H), 6.97 (d, J = 8.8 Hz, 2H), 6.84 (s, 1H), 6.67-6.66 (m, 1H), 6.52-6.50 (m, 1H), 4.44-4.35 (m, 2H), 2.70-2.68 (m, 2H), 1.18 (s, 3H), 0.82 (s, 3H).

<Synthesis Example 10. Synthesis of other compounds>

Compounds represented by compound Nos. I-S2 to S4 were synthesized by appropriately changing the compound used, solvent, and conditions in the synthesis example 9 described above.

<Measurement by ion chromatography>

(Method for preparing sample)

10.02 mg of the solid sample prepared above was weighed into a test tube, 1 mL of acetonitrile was added and extracted with ultrasonic for 20 minutes, which was held to 10 mL. A 0.45 μm membrane filter was washed with 1 mL of sample solution and the filtered sample liquid was then measured by ion chromatography (TOSOH IC2010).

(Calculation of anionic content)

The content (mass%) of each anion in the solid sample was calculated from the concentration of each anion in the sample solution. For example, as a result of measuring Compound I-S1 by ion chromatography, the Cl ion concentration in the sample solution was 94.00 μg/mL, so that the amount of Cl ions in the solid sample was calculated to be 9.40 mass%.

The molar ratio of the azole derivative to each anion was estimated by comparing the theoretical value of the anion content and the measurement value for each anion content. Note that the theoretical value of the anionic content is calculated from the estimated molar ratio of the azole derivative to each anion. When the molar ratio of the azole derivative to chlorine ion of compound I-S1 is 1:1, the theoretical value of the chlorine ion content of compound No. I-S1 is 8.7 mass%. The ion chromatography results of compound I-S1 is 9.40 mass%, and it can be seen that the molar ratio of the azole derivative to the chlorine ion of compound I-S1 is generally 1:1. The theoretical values of the anionic content for the case in which the compound No. I-S2 (sulfate salt) and the compound No. I-S3 (nitrate salt) include each anion at a molar ratio of 1:1 are 20.8 mass% and 14.4 mass%, respectively, and generally match the measurement values of the respective anions.

NMR measurement data for each of the synthesized compounds is indicated in Table 3-1 to Table 3-4, and Table 4. In addition, the salt represented by compound Nos. I-S1 to I-S3 indicates the results of ion chromatography quantification of the anionic content (mass%) in the salt in addition to the NMR measurement data.

[Table 3-1]

| Compound No. | $^1$H NMR ($CDCl_3$) |
|---|---|
| I-1 | 7.29 (d, J = 9.0 Hz, 2H), 7.20 (s, 1H), 6.96 (s, 1H), 6.91 (d, J = 9.0 Hz, 2H), 6.83 (s, 1H), 6.79 (s, 1H), 6.64 (dd, J = 8.3, 2.3 Hz, 1H), 6.33 (d, J = 8.2 Hz, 1H), 4.20 (d, J = 14.0 Hz, 1H), 3.85 (d, J = 13.9 Hz, 1H), 2.79 (d, J = 15.9 Hz, 1H), 2.70 (d, J = 15.9 Hz, 1H), 2.11 (s, 1H), 1.27 (s, 3H), 1.01 (s, 3H). |
| I-2 | 8.39 (s, 1H), 7.92 (s, 1H), 7.54 (d, J = 8.8 Hz, 1H), 7.43 (dt, J = 9.2, 4.2 Hz, 2H), 7.00 (dt, J = 9.2, 4.2 Hz, 2H), 6.86 (dd, J = 8.8, 2.8 Hz, 1H), 6.74 (d, J = 2.8 Hz, 1H), 5.83 (s, 1H), 4.34 (s, 2H), 2.72-2.67 (m, 2H), 1.89-1.72 (m, 3H), 1.55-1.48 (m, 1H). |
| I-3 | 7.89 (s, 1H), 7.71 (s, 1H), 7.27(d, J = 9.2 Hz, 2H), 6.88 (d, J = 9.2 Hz, 2H), 6.68 (s, 1H), 6.53 (d, J = 8.4 Hz, 1H), 6.46 (d, J = 8.4 Hz, 1H), 4.54 (d, J = 14.0 Hz, 1H), 4.33 (d, J - 14.0 Hz, 1H), 3.29 (s, 1H), 2.91-2.78 (m, 2H), 2.05-1.92 (m, 1H), 1.71 (dd, J = 14.0, 7.2 Hz, 1H), 1.22 (s, 3H), 0.99 (s, 3H). |
| I-4 | 7.97 (s, 1H), 7.92 (s, 1H), 7.33 (t, J = 7.5 Hz, 2H), 7.11 (t, J = 7.4 Hz, 1H), 6.97 (d, J = 7.6 Hz, 2H), 6.81 (s, 1H), 6.66 (d, J = 8.3 Hz, 1H), 6.33 (d, J = 8.2 Hz, 1H), 4.44 (d, J = 13.8 Hz, 1H), 4.25 (d, J = 13.8 Hz, 1H), 3.74 (s, 1H), 2.74 (s, 2H), 1.26 (s, 3H), 1.01 (s, 3H). |
| I-5 | 7.93 (s, 1H), 7.91 (s, 1H), 7.44 (dd, J = 8.0, 1.6 Hz, 1H), 7.24~7.19 (m, 1H), 7.10~7.06 (m, 1H), 6.95 (dd, J = 8.1, 1.5 Hz, 1H), 6.77 (d, J = 2.1 Hz, 1H), 6.60 (dd, J = 8.3, 2.3 Hz, 1H), 6.32 (d, J = 8.3 Hz, 1H), 4.44 (d, J = 13.8 Hz, 1H), 4.24 (d, J = 13.8 Hz, 1H), 3.93 (s, 1H), 2.74 (s, 2H), 1.25 (s, 3H), 1.00 (s, 3H). |
| I-6 | 7.97 (s, 1H), 7.93 (s, 1H), 7.24 (t, J = 8.2 Hz, 1H), 7.08~7.05 (m, 1H), 6.95~6.94 (m, 1H), 6.86~6.82 (m, 2H), 6.67 (dd, J = 8.2, 2.3 Hz, 1H), 6.35 (d, J = 8.2 Hz, 1H), 4.45 (d, J = 13.8 Hz, 1H), 4.26 (d, J = 13.8 Hz, 1H), 3.84 (s, 1H), 2.76 (s, 2H), 1.26 (s, 3H), 1.02 (s, 3H). |
| I-7 | 7.97 (s, 1H), 7.92 (s, 1H), 7.26 (d, J = 8.8 Hz, 2H), 6.90 (d, J = 8.8 Hz, 2H), 6.89 (s, 1H), 6.64 (d, J = 7.8 Hz, 1H), 6.33 (d, J = 7.8 Hz, 1H), 4.42 (d, J = 14.8 Hz, 1H), 4.19 (d, J = 14.8 Hz, 1H), 3.76 (s, 1H), 2.75 (s, 2H), 1.26 (s, 3H), 1.01 (s, 3H). |
| I-8 | 7.96 (s, 1H), 7.91 (s, 1H), 7.45 (d, J = 2.5 Hz, 1H), 7.19 (dd, J = 8.8, 2.5 Hz, 1H), 6.88 (d, J = 8.8 Hz, 1H), 6.77 (s, 1H), 6.60 (d, J = 8.2 Hz, 1H), 6.33 (d, J = 8.3 Hz, 1H),4.44 (d, J = 13.8 Hz, 1H), 4.24 (d, J = 13.8 Hz, 1H), 3.85 (s, 1H), 2.75 (s, 2H), 1.26 (s, 3H), 1.00 (s, 3H). |
| I-9 | 7.98 (s, 1H), 7.93 (s, 1H), 7.37 (d, J = 8.8 Hz, 1H), 7.04 (d, J = 2.8 Hz, 1H), 6.83~6.80 (m, 2H), 6.66 (dd, J = 8.2, 2.3 Hz, 1H), 6.36 (d, J = 8.2 Hz, 1H), 4.45 (d, J = 13.8 Hz, 1H), 4.25 (d, J = 13.8 Hz, 1H), 3.85 (s, 1H), 2.77 (s, 2H), 1.27 (s, 3H), 1.02 (s, 3H). |

[Table 3-2]

| Compound No. | $^1$H NMR ($CDCl_3$) |
|---|---|
| I-10 | 7.97 (s, 1H), 7.92 (s, 1H), 7.42 (d, J = 8.9 Hz, 2H), 6.85 (d, J = 8.9 Hz, 2H), 6.79 (s, 1H), 6.65~6.64 (m, 1H), 6.34 (d, J = 8.2 Hz, 1H), 4.44 (d, J = 13.8 Hz, 1H), 4.25 (d, J = 13.8 Hz, 1H), 3.77 (s, 1H), 2.75 (s, 2H), 1.26 (s, 3H), 1.01 (s, 3H). |
| I-11 | 7.96 (s, 1H), 7.92 (s, 1H), 7.04~7.00 (m, 2H), 6.96~6.93 (m, 2H), 6.76 (s, 1H), 6.61 (d, J = 8.3 Hz, 1 H), 6.31 (d, J = 8.3 Hz, 1H), 4.43 (d, J = 13.8 Hz, 1H), 4.24 (d, J = 13.8 Hz, 1H), 3.77 (s, 1H), 2.73 (s, 2H), 1.25 (s, 3H), 1.00 (s, 3H). |
| I-12 | 7.97 (s, 1H), 7.92 (s, 1H), 7.14~7.07 (m, 1H), 6.82~6.77 (m, 2H), 6.70~6.68 (m, 1H), 6.64 (dd, J = 8.2, 2.3 Hz, 1H), 6.34 (d, J = 8.2 Hz, 1H), 4.44 (d, J = 13.8 Hz, 1H), 4.25 (d, J = 13.8 Hz, 1H), 3.82 (s, 1H), 2.75 (s, 2H), 1.26 (s, 3H), 1.01 (s, 3H). |

(continued)

| Compound No. | $^1H$ NMR ($CDCl_3$) |
|---|---|
| I-13 | 7.95 (s, 1H), 7.91 (s, 1H), 7.17 (d, J = 8.3 Hz, 2H), 6.98~6.95 (m, 2H), 6.81 (s, 1H), 6.65 (dd, J = 8.2, 2.3 Hz, 1H), 6.34 (d, J = 8.2 Hz, 1H), 4.44 (d, J = 13.8 Hz, 1H), 4,26 (d, J = 13,8 Hz, 1H), 4.06 (s, 1H), 2.75 (s, 2H), 1.26 (s, 3H), 1.00 (s, 3H). |
| I-14 | 7.98 (s, 1H), 7.94 (s, 1H), 7.57 (d, J = 8.6 Hz, 2H), 7.00 (d, J = 8.5 Hz, 2H), 6.86 (s, 1H), 6.70 (dd, J = 8.2, 2.2 Hz, 1H), 6.38 (d, J = 8.2 Hz, 1H), 4.46 (d, J = 13.8 Hz, 1H), 4.26 (d, J = 13.8 Hz, 1H), 3.86 (s, 1H), 2.77 (s, 2H), 1.27 (s, 3H), 1.02 (s, 3H). |
| I-15 | 8.11 (d, J = 2.0 Hz, 1H), 7.97 (s, 1H), 7.93 (s, 1H), 7.29-7.23 (m, 2H), 6.82 (s, 1H), 6.67 (dd, J = 8.2, 2.3 Hz, 1H), 6.39 (d, J = 8.3 Hz, 1H), 4.45 (d, J = 13.8 Hz, 1H), 4.25 (d, J = 13.8 Hz, 1H), 3.88 (s, 1H), 2.76 (s, 2H), 1.27 (s, 3H), 1.01 (s, 3H). |
| I-16 | 8.11 (d, J = 2.7 Hz, 1H), 7.97 (s, 1H), 7.95 (s, 1H), 7.63 (dd, J = 8.7, 2.7 Hz, 1H), 6.95 (s, 1H), 6.83 (d, J = 8.7 Hz, 1H), 6.76 (dd, J = 8.2, 2.2 Hz, 1H), 6.40 (d, J = 8.2 Hz, 1H), 4.45 (d, J = 13.8 Hz, 1H), 4.27 (d, J = 13.8 Hz, 1H), 3.85 (s, 1H), 2.79 (s, 2H), 1.26 (s, 3H), 1.02 (s, 3H). |
| I-17 | 7.95 (3, 1H), 7.91 (s, 1H), 7.29 (d, J = 8.7 Hz, 2H), 6.92 (d, J = 8.8 Hz, 2H), 6.79 (s, 1H), 6.73 (d, J = 8.3 Hz, 1H), 6.50 (d, J = 8.3 Hz, 1H),4.32 (d, J = 14.0 Hz, 1H), 4.28 (d, J = 14.2 Hz, 1H), 3.39 (s, 1H), 3.12 (d, J = 16.3 Hz, 1H), 2.55 (d, J = 16.4 Hz, 1H), 1.11~1.06 (m, 1H), 0.82~0.77 (m, 1H), 0.73~0.67 (m, 1H), 0.43~0.38 (m, 1H). |
| I-18 | 8.47 (s, 1H), 8.04 (s, 1H), 7.95 (d, J = 8.6 Hz, 1H), 7.66 (s, 1H), 7.40 (d, J = 8.3 Hz, 1H), 7.14~7.07 (m, 3H), 4.47 (m, 1H), 4.28 (d, J = 12.1 Hz, 1H), 3.48 (s, 1H), 2.89 (d, J = 16.2 Hz, 1H), 2.80 (d, J = 16.2 Hz, 1H), 1.33 (s, 3H), 0.75 (s, 3H). |

[Table 3-3]

| Compound No. | $^1H$ NMR ($CDCl_3$) |
|---|---|
| I-19 | 8.51 (s, 2H), 8.02 (s, 1H), 7.67 (s, 1H), 7.41 (d, J = 8.3 Hz, 1H), 7.16 (s, 1H), 7.13 (d, J = 8.3 Hz, 1H), 4.48 (dd, J = 12.1, 8.2 Hz, 1H), 4.26 (d, J = 12.1 Hz, 1H), 3.53 (br, 1H), 2.90 (d, J = 16.2 Hz, 1H), 2.80 {d, J = 16.2 Hz, 1H), 1.33 (s, 3H), 0.75 (s, 3H). |
| I-20 | 8.40-8.37 (m, 2H), 8.03 (s, 1H), 7.66 (s, 1H), 7.42 (d, J = 8.3 Hz, 1H), 7.21 (dd, J = 7.9, 4.9 Hz, 1H), 7.18 (s, 1H), 7.14 (dd, J = 8.3, 2.3 Hz, 1H), 4.47 (dd, J = 12.2, 8.7 Hz, 1H), 4.29 (dd, J = 12.2, 5.5 Hz, 1H), 3.49 (dd, J = 8.7, 5.6 Hz, 1H), 2.90 (d, J = 16.2 Hz, 1H), 2.80 (d, J = 16.2 Hz, 1H), 1.33 (s, 3H), 0.76 (s, 3H). |
| I-21 | 8.03 (s, 1H), 7.67 (s, 1H), 7.54 (d, J = 9.1 Hz, 1H), 7.40 (d, J = 8.3 Hz, 1H), 7.24-7.21 (m, 2H), 7.14 (dd, J = 8.3, 2.3 Hz, 1H), 4.47 (dd, J = 12.2, 8.5 Hz, 1H), 4.26 (dd, J = 12.2, 5.6 Hz, 1H), 3.43 (dd, J = 8.6, 5.8 Hz, 1H), 2.88 (d, J = 16.2 Hz, 1H), 2.79 (d, J = 16.2 Hz, 1H), 1.32 (s, 3H), 0.74 (s, 3H). |
| I-22 | 8.35 (dd, J = 5.0, 1.9 Hz, 1H), 8.05-8.03 (m, 2H), 7.65 (s, 1H), 7.41 (d, J = 8.3 Hz, 1H), 7.19-7.13 (m, 3H), 4.47 (dd, J = 12.2, 8.7 Hz, 1H), 4.29 (dd, J = 12.2, 5.5 Hz, 1H), 3.49 (dd, J = 8.6, 5.5 Hz, 1H), 2.90 (d, J = 16.2 Hz, 1H), 2.80 (d, J = 16.2 Hz, 1H), 1.33 (s, 3H), 0.76 (s, 3H). |
| I-23 | 8.50 (d, J = 2.8 Hz, 1H), 8.04 (s, 1H), 7.69 (dd, J = 8.6, 0.6 Hz, 1H), 7.64 (s, 1H), 7.45 (d, J = 8.2 Hz, 1H), 7.36 (dd, J = 8,6, 2.8 Hz, 1H), 7.03 (m, 2H), 4.52 (dd, J = 12.1, 8.1 Hz, 1H), 4.23 (dd, J = 12.1, 5.9 Hz, 1H), 3.32 (t, J = 7.8Hz, 1H), 2.89 (d, J = 16.4 Hz, 1H), 2.79 (d, J = 16.3 Hz, 1H), 1.31 (s, 3H), 0.75 (s, 3H) |
| I-24 | 7.97 (s, 1H), 7.93 (s, 1H), 7.30 (t, J = 8.6 Hz, 1H), 6.83 (d, J = 2,0 Hz, 1H), 6.75 (dd, J = 10.1, 2.7 Hz, 1H), 6.72-6.66 (m, 2H), 6.36 (d, J = 8.0 Hz, 1H), 4.45 (d, J = 13.8 Hz, 1H), 4.35 (d, J = 13.8 Hz, 1H), 3.87 (s, 1H), 2,76 (s, 2H), 1.27 (s, 3H), 1.01 (s, 3H). |
| I-25 | 7.98 (s, 1H), 7.93 (s, 1H), 7.59 (dd, J = 6.8, 2.1 Hz, 2H), 6.96 (dd, J = 6.9, 2.1 Hz, 2H), 6.88 (s, 1H), 6.71 (dd, J = 8.2, 2.3 Hz, 1H), 6.40 (d, J = 8.2 Hz, 1H), 4.46 (d, J = 13.8 Hz, 1H), 4.26 (d, J = 13.8 Hz, 1H), 3.89 (s, 1H), 2.79 (s, 2H), 1.28 (s, 3H), 1.03 (s, 3H). |

(continued)

| Compound No. | $^1H$ NMR ($CDCl_3$) |
|---|---|
| I-26 | 8.20 (dd, J = 7.1, 2.2 Hz, 2H), 7.99 (s, 1H), 7.94 (s, 1H), 6.97 (dd, J = 7.1,2.2 Hz, 2H), 6.91 (s, 1H), 6.74 (dd, J = 8.2, 2.2 Hz, 1H), 6.42 (d, J = 8.2 Hz, 1H), 4.47 (d, J = 13.8 Hz, 1H), 4.27 (d, J = 13.8 Hz, 1H), 3.91 (s, 1H), 2.80 (s, 2H), 1.29 (s, 3H), 1.04 (s, 3H). |

[Table 3-4]

| Compound No. | $^1H$ NMR ($CDCl_3$) |
|---|---|
| I-27 | 8.04 (s, 1H), 8.01 (s, 1H), 7.29 (dd, J = 6.7, 2.1 Hz, 2H), 6.92 (dd, J = 6.8, 2.2 Hz, 2H), 6.85-6.80 (m, 3H), 4.38 (s, 2H), 3.71 (s, 1H), 3.01-2.95 (m, 1H), 2.83-2.75 (m, 1H), 2.38-2.32 (m, 1H), 2.17-2.09 (m, 1H). |
| I-28 | 7.96 (s, 1H), 7.93 (s, 1H), 7.61 (d, J = 8.9 Hz, 2H), 6.70 (s, 1H), 6.73 (d, J = 8.8 Hz, 2H), 6.65 (dd, J = 8.2, 2.2 Hz, 1H), 6.34 (d, J = 8.2 Hz, 1H), 4.44 (d, J = 13.8 Hz, 1H), 4.25 (d, J = 13.8 Hz, 1H), 3.83 (s, 1H), 2.74 (s, 2H), 1.26 (s, 3H), 1.00 (s, 3H). |
| I-29 | 7.96 (s, 1H), 7.82 (s, 1H), 7.28-7.26 (m, 2H), 6.88 (dd, J = 6.8 Hz, 2H), 6.75 (s, 1H), 6.58 (d, J = 6.0 Hz, 1H), 6.14 (d, J = 8.2 Hz, 1H), 4.53 (d, J = 13.6 Hz, 1H), 4.24 (d, J = 13.7 Hz, 1H), 3.84 (s, 1H), 2.83 (d, J = 16.2 Hz, 1H), 2.56 (d, J = 16.1 Hz, 1H), 1.84-1.79 (m, 1H), 1.74-1.69 (m, 2H), 1.27-1.21 (m, 1H), 1.10 (t, J = 7.4 Hz, 3H), 0.80 (t, J = 7.4 Hz, 3H). |
| I-30 | 7.96 (s, 1H), 7.82 (s, 1H), 7.29-7.26 (m, 2H), 6.89 (dd, J = 6.7, 2,2 Hz, 2H), 6.74 (s, 1H), 6.57 (d, J = 6.1 Hz, 1H), 6.12 (d, J = 8.2 Hz, 1H),4.48 (d, J = 13.6 Hz, 1H), 4.22 (d, J = 13.6 Hz, 1H), 3.87 (s, 1H), 2.84 (d, J = 16.2 Hz, 1H), 2.58 (d, J = 16.2 Hz, 1H), 1.68-1.59 (m, 4H), 1.55-1.45 (m, 1H), 1.25-1.13 (m, 3H), 0.97 (t, J = 6.8 Hz, 3H), 0.84 (t, J = 6.8 Hz, 3H). |
| I-31 | 7.96 (s, 1H), 7.89 (s, 1H), 7.03 (td, J = 9.0, 5.5 Hz, 1H), 6.96-6.91 (m, 1H), 6.87-6.82 (m, 1H), 6.74 (d, J = 2.2 Hz, 1H), 6.58 (dd, J = 8.8, 2.2 Hz, 1H), 6.30 (d, J = 8.3 Hz, 1H), 4.43 (d, J = 13.8 Hz, 1H), 4.23 (d, J = 13.8 Hz, 1H), 3.77 (s, 1H), 2.73 (s, 2H), 1.25 (s, 3H), 1.00 (s, 3H). |
| I-32 | 8.01 (s, 1H), 7.85 (s, 1H), 7.27 (dd, J = 6.5, 2.4 Hz, 2H), 6.89 (dd, J = 6.9, 2.1 Hz, 2H), 6.80 (d, J = 2.1 Hz, 1H), 6.64 (dd, J = 8.3, 2.8 Hz, 1H), 6.25 (d, J = 8.3 Hz, 1H), 4.42 (d, J = 13.8 Hz, 1H), 4.13-4.11 (m, 2H), 3.08-3.04 (m, 1H), 2 64-2.61 (m, 1H), 2.51 (dd, J = 15.8, 10.3 Hz, 1H), 1.29 (d, J = 6.9 Hz, 3H). |
| I-33 | 8.10 (s, 1H), 7.99 (s, 1H), 7.29 (dd, J = 6.2, 2.1 Hz, 2H), 6.95-6.92 (m, 3H), 6.83-6.82 (m, 2H), 4.47 (d, J = 14.5 Hz, 1H),4.37 (d, J = 13.8 Hz, 1H), 3.29 (s, 1H), 3.03-2.99 (m, 1H), 2.61-2.58 (m, 1H), 2.47-2.44 (m, 1H), 0.90 (d, J = 6.9 Hz, 3H). |
| I-34* | 10.35 (s, 1H), 8.27 (s, 1H), 7.86 (s, 1H), 7.29 (d, J = 9.0 Hz, 1H), 6.83 (d, J = 2.1 Hz, 1H), 6.67 (dd, J = 8.3, 2.8 Hz, 1H), 6.56 (d, J = 2.8 Hz, 1H), 6.51 (d, J = 7.6 Hz, 1H), 6.40 (dd, J = 9.0, 2.8 Hz, 1H), 5.35 (s, 1H), 4.44 (d, J = 14.5 Hz, 1H), 4.36 (d, J = 14.5 Hz, 1H), 2.71 (d, J = 15.8 Hz, 1H), 2.67 (d, J = 15.8 Hz, 1H), 1.18 (s, 3H), 0.81 (s, 3H). |
| I-35 | 8.01 (s, 1H), 7.82 (s, 1H), 7.27 (dd, J = 6.9, 2.1 Hz, 2H), 6.88 (dd, J = 6.9, 2.1 Hz, 2H), 6.80 (d, J = 2.8 Hz, 1H), 6.63 (dd, J = 8.3, 2.1 Hz, 1H), 6.19 (d, J = 8.3 Hz, 1H), 4.43 (d, J = 13.8 Hz, 1H), 4.13 (s, 1H), 4.10 (d, J = 13.8 Hz, 1H), 3.14-3.09 (m, 1H), 2.49-2.41 (m, 2H), 1.98-1.94 (m, 1H), 1.52-1.46 (m, 1H), 1.10 (t, J = 7.6 Hz, 3H). |
| I-36 | 8.10 (s, 1H), 7.99 (s, 1H), 7.29 (dd, J = 6.2, 2.1 Hz, 2H), 6.93 (dd, J = 6.9, 2.1 Hz, 2H), 6.90 (d, J = 8.3 Hz, 1H), 6.82-6.81 (m, 2H), 4.48 (d, J = 13.8 Hz, 1H), 4.40 (d, J = 14.5 Hz, 1H), 3.24 (s, 1H), 3.03-2.99 (m, 1H), 2.69 (dd, J = 16.5, 5.5 Hz, 1H), 2.22-2.19 (m, 1H), 1.35-1.31 (m, 1H), 1.26-1.20 (m, 1H), 0.92 (t, J = 7.2 Hz, 3H). |
| I-37 | 8.01 (s, 1H), 7.81 (s, 1H), 7.28-7.27 (m, 2H), 6.88 (dd, J = 6.9, 2.1 Hz, 2H), 6.80 (d, J = 2.1 Hz, 1H), 6.62 (dd, J = 8.3, 2.1 Hz, 1H), 6.18 (d, J = 8.3 Hz, 1H), 4.43 (d, J = 13.8 Hz, 1H), 4.15 (s, 1H), 4.10 (d, J = 13.8 Hz, 1H), 3.09 (dd, J = 15.0, 6.6 Hz, 1H), 2.55-2.45 (m, 2H), 1.89-1.85 (m, 1H), 1.59-1.56 (m, 1H), 1.48-1.42 (m, 2H), 1.01 (t, J = 6.9 Hz, 3H). |

(continued)

| Compound No. | $^1H$ NMR ($CDCl_3$) |
|---|---|
| I-38 | 8.10 (s, 1H), 7.99 (s, 1H), 7.29 (dd, J = 6.5, 2.4 Hz, 2H), 6.93 (dd, J = 6.5, 2.4 Hz, 2H), 6.91-6.90 (m, 1H), 6.82-6.80 (m, 2H), 4.49 (d, J = 13.8 Hz, 1H), 4.41 (d, J = 13.8 Hz, 1H), 3.22 (s, 1H), 3.00 (dd, J = 16.5, 7.6 Hz, 1H), 2.68 (dd, J = 16.2, 5.9 Hz, 1H), 2.31-2.27 (m, 1H), 1.42-1.37 (m, 1H), 1.28-1.18 (m, 3H), 0.88 (t, J = 6.9 Hz, 3H). |
| * For only I-34, DMSO-d 6 is used instead of $CDCl_3$. | |

[Table 4]

| Compound No. | Ion chromatography | $^1H$ NMR DMSO-$d_6$ |
|---|---|---|
| I-S1 | Cl amount: 9.40% | 8.39 (s, 1H), 7.94 (s, 1H), 7.42 (d, J = 8.8 Hz, 2H), 6.97 (d, J = 8.8 Hz, 2H), 6.84 (s, 1H), 6.67-6.66 (m, 1H), 6.52-6.50 (m, 1H), 4.44-4.35 (m, 2H), 2.70-2.68 (m, 2H), 1.18 (s, 3H), 0.82 (s, 3H). |
| I-S2 | $SO_4$ amount: 20.60% | 8.36 (s, 1H), 7.92 (s, 1H), 7.42 (d, J = 8.8 Hz, 2H), 6.97 (d, J = 8.8 Hz, 2H), 6.84 (s, 1H), 6.68-6.67 (m, 1H), 6.52-6.49 (m, 1H), 4.45 (d, J = 14.4 Hz, 1H), 4.36 (d, J = 14.4 Hz, 1H), 2.71-2.68 (m, 2H), 1.18 (s, 3H), 0.82 (s, 3H). |
| I-S3 | $NO_3$ amount: 15.00% | 8.44 (s, 1H), 7.98 (s, 1H), 7.42 (d, J = 6.8 Hz, 2H), 6.98 (d, J = 6.8 Hz, 2H), 6.84 (d, J = 2.0 Hz, 1H), 6.68 (dd, J = 8.0, 2.0 Hz, 1H), 6.50 (d, J = 8.0 Hz, 1H), 4.46 (d, J = 14.0 Hz, 1H), 4.37 (d, J = 14.0 Hz, 1H), 2.74 (d, J = 15.6 Hz, 1H), 2.66 (d, J = 15.6 Hz, 1H), 1.18 (s, 3H), 0.82 (s, 3H) |
| I-S4 | - | 8.40 (s, 1H), 7.96 (s, 1H), 7.48-7.41 (m, 4H), 7.11 (d, J = 8.4 Hz, 2H), 6.97 (d, J = 8.8 Hz, 2H), 6.84 (s, 1H), 6.67-6.65 (m, 1H), 6.51-6.49 (m, 1H), 4.45-4.38 (m, 2H), 2.72-2.67 (m, 2H), 2.29 (s, 3H), 1.18 (s, 3H), 0.82 (s, 3H). |

<Formulation Example>

Hydrates and emulsions were formulated as follows using any synthesized azole derivative described in this patent.

Formulation Example 1 (Wettable powder)

Azole derivative 20.0 parts

Sodium lauryl sulfate 2 parts

Sodium salt of alkyl naphthalene sulfonic acid formalin condensate 5 parts

Zinc stearate 0.2 parts

White Carbon 3 parts

Clay 69.8 parts

The above components were pulverized and mixed to form a wettable powder.

Formulation Example 2 (Emulsion)

Azole derivative 2.0 parts
Polyoxyalkylene alkyl ether/alkylbenzene sulfonic acid metal salt/alkylbenzene mixture 15.0 parts
Methyl 5-(dimethylamino)-2-methyl-5-oxopentanoate 12.5 parts
N,N-dimethyloctaneamide/N,N-dimethyldecaneamide mixture 37.3 parts
Solvent naphtha 33.2 parts

The above components were uniformly mixed and dissolved, to result in an emulsion.

<Test Example 1: Antimicrobial activity test for plant pathogens>

The antimicrobial activity of the compound according to an embodiment of the present invention against various plant pathogenic filamentous fungi was tested by a Petri dish test.

The compound of the present invention was dissolved in dimethyl sulfoxide and added to a PDA culture medium (potato dextrose agar medium) cooled to around 60°C after autoclave sterilization to a predetermined chemical concentration, and 1% (V/V) was added to the PDA culture medium. The culture medium was poured into the Petri dish and a plate culture medium containing the compound of the present invention was prepared by mixing well so that the chemical concentration in the PDA culture medium was uniform.

On the other hand, the plugs of mycelium of various plant pathogens previously cultured on the PDA culture medium was punched out using a cork borer having a diameter of 4 mm, and inoculated into the aforementioned chemical containing plate culture medium. A diameter of mycelial colony on the chemical-treated plate was measured after culturing at temperature for a predetermined period of time according to Table 5. The fungal growth inhibition rate (%) was calculated by the following formula in comparison with a diameter of the mycelial colony on the untreated plate containing no chemical.

$$R = 100\ (dc - dt)/dc$$

(wherein R = % fungal growth inhibition rate (%), dc = diameter of mycelial colony on untreated plate, dt = diameter of mycelial colony on chemical-treated plate)
The obtained results were evaluated for five steps according to the criteria indicated in Table 6. The larger the antimicrobial activity index, the better the antimicrobial action.

[Table 5]

| Abbreviations | Fungal Name | Culture temperature (°C) | Culture days (days) |
|---|---|---|---|
| *Z. t* | *Zymoseptoria tritici* | 25 | 14 |
| *F. g* | *Fusarium graminearum* | 25 | 3 |
| *R. s* | *Rhynchosporium secalis* | 25 | 14 |

[Table 6]

| Fungal growth inhibition rate | Antimicrobial activity index |
|---|---|
| 80% or more | 5 |
| Less than 80% and 60% or more | 4 |
| Less than 60% and 40% or more | 3 |
| Less than 40% and 20% or more | 2 |
| Less than 20% | 1 |

Test Example A: Zymoseptoria tritici

An antibacterial test was performed using Zymoseptoria tritici according to the aforementioned method. The compounds 1-1, I-3 to 1-13, 1-15 to I-29, I-31 to I-35, I-37 and I-38 all showed antibacterial index of 5 at 100 mg/L of

the test substance.

Test Example B: Fusarium graminearum

An antibacterial test was performed using Fusarium graminearum by the method described above. The compounds 1-1, I-3 to 1-13, 1-15 to 1-17, I-24 to I-28, 1-31 to I-35, I-37 and I-38 all showed antibacterial index of 5 at 100 mg/L of the test substance.

Test Example C: Rhynchosporium secalis

An antibacterial test was performed using Rhynchosporium secalis according to the aforementioned method. The compounds 1-1, I-3 to 1-13, 1-15 to 1-17, I-24 to I-29, 1-31 to I-35, I-37 and I-38 all showed antibacterial index of 5 at 100 mg/L of the test substance.

<Test Example 2: Cucumber gray mold disease controlling effect test>

The compound of the present invention was dissolved in acetone, 0.5% (V/V) thereof added to water so that the concentration became 100 g/ha, and cucumbers in the cotyledon stage (variety: Hanjiro Fushinari) cultivated using a square plastic pot (6.5 cm × 6.5 cm) were sprayed at a ratio of 1,000 L/ha. After the sprayed leaves were air-dried, a paper disc (8 mm in diameter) impregnated with spore suspension of Botrytis cinerea was placed and kept under high-humidity conditions of 20°C. After inoculation, a disease spot area ratio of cucumber gray mold was investigated on day 4 and a controlling rate was calculated according to the following formula.

$$\text{Controlling rate (\%)} = (1 - \text{average incidence rate in sprayed section/average incidence rate in non-sprayed section}) \times 100$$

The incidence rate was also determined based on Table 7 below.

[Table 7]

| Incidence rate | Disease spot area ratio (%) |
|---|---|
| 0 | 0 |
| 0.1 | <5 |
| 0.5 | <10 |
| 1 | <20 |
| 2 | <40 |
| 3 | <60 |
| 4 | <80 |
| 5 | 100 |

In the above tests, all of the compounds I-4, I-6 to I-8, 1-10 to 1-12, I-27, I-28, I-32, I-33, I-35, I-37 and I-S1 to I-S4 exhibited a controlling rate of 80% or higher.

<Test Example 3: Cucumber downy mildew disease controlling effect test>

The compound of the present invention was dissolved in acetone, 0.5% (V/V) thereof added to water so that the concentration became 100 g/ha, and cucumbers in the cotyledon stage (variety: Hanjiro Fushinari) cultivated using a square plastic pot (6.5 cm × 6.5 cm) were sprayed at a ratio of 1,000 L/ha. The leaves were air-dried and then spray-inoculated with spore suspension (adjusted to $1 \times 10^5$ cells/ml) of Pseudoperonospora cubensis and kept at 20°C under the high-humidity conditions. After inoculation, the disease spot area ratio of cucumber downy mildew was investigated on day 7, and the controlling rate was calculated in the same manner as in Test Example 2.

In the above tests, both the compounds I-17 and I-30 exhibited a controlling rate of 80% or higher.

<Test Example 4: Wheat powdery mildew disease controlling effect test

Wheat (variety: Norin No. 61) was cultivated from the first to second leaf stage using a square plastic pot (6.5 cm × 6.5 cm). It was dissolved in acetone, 0.5% (V/V) thereof was added to water so that a concentration became 100 g/ha, and the wheat was sprayed at a ratio of 1,000 L/ha. A dispersion on the plant body was air-dried, and then the spore of Erysiphe graminis f. sp tritici from wheat powdery mildew seedlings was sprinkled on and inoculated. After that, it was managed in a greenhouse. On day 7 to day 14 after inoculation, the disease spot area ratio of wheat powdery mildew was investigated, and the controlling rate was calculated in the same manner as in Test Example 2.

In the above tests, all of the compounds I-4, I-7, I-10, I-15, I-17, I-24, I-27 to I-29, I-32, I-33 and I-35 to I-37 exhibited a controlling rate of 80% or higher.

<Test Example 5: Wheat brown rust disease controlling effect test>

Wheat (variety: Norin No. 61) was cultivated from the first to second leaf stage using a square plastic pot (6.5 cm × 6.5 cm). The compound of the present invention was dissolved in acetone, 0.5% (V/V) was added to water so that a concentration became 100 g/ha, and the wheat was sprayed at a ratio of 1,000 L/ha. After the dispersion on the plant body was air-dried, the spore of Puccinia recondita (adjusted to 200 pieces/field of view, added Gramin S to 60 ppm) was spray-inoculated, and kept at 20°C under the high-humidity conditions for 24 hours. After that, it was managed in a greenhouse. On day 10 to day 14 after inoculation, the disease spot area ratio of wheat brown rust was investigated, and the controlling rate was calculated in the same manner as in Test Example 2.

In the above tests, all of the compounds I-7, I-10, I-12, I-15 to I-17, I-24, I-27, I-32, I-33, I-35 to I-38 and I-S1 to I-S4 exhibited a controlling rate of 80% or higher.

<Test Example 6: Wheat septoria leaf blotch disease controlling effect test>

Wheat (variety: Norin No. 61) was cultivated from the first to second leaf stage using a square plastic pot (6.5 cm × 6.5 cm). The compound of the present invention was dissolved in acetone, 0.5% (V/V) was added to water so that a concentration became 100 g/ha, and the wheat was sprayed at a ratio of 1,000 L/ha. After the dispersion on the plant body was air-dried, the spore of Zymoseptoria tritici (adjusted to $1 \times 10^7$/mL, added Gramin S to 60 ppm) was spray-inoculated, and kept at 20°C under the high-humidity conditions for 72 hours. Subsequently, it was managed in an artificial weather chamber. On day 27 to day 33 after inoculation, the disease spot area ratio of wheat leaf blight was investigated, and the controlling rate was calculated in the same manner as in Test Example 2.

In the above tests, all of the compounds I-1, I-5, I-7, I-10 to I-12, I-15 to I-17, I-24, I-25, I-27 to I-29, I-31 to I-33, I-35, I-36, and I-S1 to I-S4 exhibited a controlling rate of 80% or higher.

<Test Example 7: Asian soybean rust disease controlling effect test>

Soybean (variety: Enrei) was cultivated from the first to second leaf stage using a square plastic pot (6.5 cm × 6.5 cm). The compound of the present invention was dissolved in acetone, 0.5% (V/V) was added to water so that a concentration became 100 g/ha, and the soybean was sprayed at a ratio of 1,000 L/ha. After the dispersion on the plant body was air-dried, the spore of Phakopsora pachyrhizi (adjusted to $1 \times 10^6$/mL, added Gramin S to 60 ppm) was spray-inoculated, and kept at 25°C under the high-humidity conditions for 24 hours. After that, it was managed in a greenhouse. On day 15 to 20 after inoculation, a disease spot area ratio of Asian soybean rust was investigated, and a controlling rate was calculated according to the following formula.

$$\text{Controlling rate (\%)} = (1 - \text{average incidence rate in sprayed section/average incidence rate in non-sprayed section}) \times 100$$

The incidence rate was also determined based on Table 8 below.

[Table 8]

| Incidence rate | Disease spot area ratio (%) |
|---|---|
| 0 | 0 |
| 0.5 | <5 |

(continued)

| Incidence rate | Disease spot area ratio (%) |
|---|---|
| 1 | <10 |
| 2 | <25 |
| 3 | <50 |
| 4 | <100 |
| 5 | 100 |

In the above tests, all of the compounds I-4 to I-7, I-9 to I-13, I-15 to I-17, I-24, I-25, I-28, I-31 to I-33, I-35 to I-38, and I-S1 to I-S4 exhibited a controlling rate of 80% or higher.

[Industrial Applicability]

The azole derivative according to an embodiment of the present invention can be suitably used as an active ingredient of an agricultural and horticultural fungicide and an industrial material protectant.

## Claims

1. An azole derivative represented by general formula (I) below, or an N-oxide or acceptable salt thereof,

[Chem. 1]

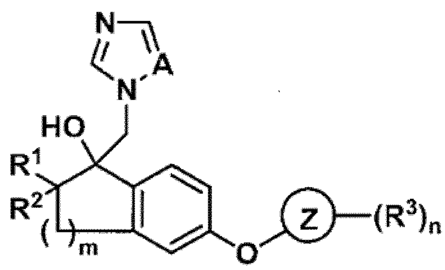

(I)

wherein A is N or CH;
$R^1$ and $R^2$ are each independently hydrogen, a $C_1$-$C_6$-alkyl group, a $C_3$-$C_8$-cycloalkyl group, or a $C_3$-$C_8$-cycloalkyl-$C_1$-$C_4$-alkyl group;
$R^1$ and $R^2$ may be bonded to each other to form a ring;
Z is a phenyl group or a 5- or 6-membered aromatic heterocyclic ring containing 1, 2, 3, or 4 heteroatoms selected from O, N, and S;
$R^3$ is halogen, a hydroxy group, an amino group, a nitrile group, a nitro group, a pentafluorosulfanyl group, a $C_1$-$C_4$-alkyl group, a $C_1$-$C_4$-haloalkyl group, a $C_1$-$C_4$-alkoxy group, or a $C_1$-$C_4$-haloalkoxy group;
$R^3$ is bonded to any substitution position of Z in a number of n;
n is 0, 1, 2, 3, 4, or 5; and
m is 1 or 2.

2. The azole derivative, or the N-oxide or acceptable salt thereof according to claim 1, wherein m is 1 in the general formula (I).

3. The azole derivative, or the N-oxide or acceptable salt thereof according to claim 1 or 2, wherein Z is a phenyl group in the general formula (I).

4. A method for producing the azole derivative according to claim 1, comprising:

producing the azole derivative by reacting a compound represented by general formula (II) below with the coexistence of 1,2,4-triazole or imidazole or an alkali metal salt thereof and sulfur ylide,

[Chem. 2]

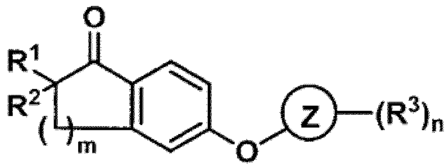

(II)

wherein $R^1$, $R^2$, $R^3$, Z, m, and n are the same as $R^1$, $R^2$, $R^3$, Z, m, and n in formula (I) respectively.

**5.** An agricultural or horticultural chemical or industrial material protectant comprising: the azole derivative described in any one of claims 1 to 3 as an active ingredient.

| INTERNATIONAL SEARCH REPORT | International application No. PCT/JP2021/008690 |
|---|---|

A. CLASSIFICATION OF SUBJECT MATTER
Int.Cl. C07D233/56(2006.01)i, A01N43/50(2006.01)i, A01N43/653(2006.01)i, A01N47/02(2006.01)i, A01P1/00(2006.01)i, A01P3/00(2006.01)i, C07D249/08(2006.01)i,C07D401/12(2006.01)i,C07D403/12(2006.01)i
FI: C07D233/56CSP, A01N43/50C, A01N43/653C, A01N47/02, A01P1/00, A01P3/00, C07D249/08514, C07D401/12, C07D403/12
According to International Patent Classification (IPC) or to both national classification and IPC

B. FIELDS SEARCHED
Minimum documentation searched (classification system followed by classification symbols)
Int.Cl. C07D233/56, A01N43/50, A01N43/653, A01N47/02, A01P1/00, A01P3/00, C07D249/08, C07D401/12, C07D403/12

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 62-230771 A (HOECHST AKTIENGESELLSCHAFT) 09 October 1987 (1987-10-09), claims, formula (II), page 18, upper right column to page 19, lower right column, page 23, upper right column, examples, table 3, compound no. 11, 21, organism experimental example B.) plant protection | 1-5 |
| A | WO 2019/093522 A1 (KUREHA CORPORATION) 16 May 2019 (2019-05-16), entire text | 1-5 |
| A | JP 2014-520832 A (BASF SE) 25 August 2014 (2014-08-25), entire text | 1-5 |
| A | JP 54-027563 A (ROHM AND HAAS CO.) 01 March 1979 (1979-03-01), entire text | 1-5 |
| A | JP 11-080127 A (KUREHA CHEMICAL IND CO., LTD.) 26 March 1999 (1999-03-26), entire text | 1-5 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 April 2021 | 27 April 2021 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2021/008690

C (Continuation). DOCUMENTS CONSIDERED TO BE RELEVANT

| Category* | Citation of document, with indication, where appropriate, of the relevant passages | Relevant to claim No. |
|---|---|---|
| A | JP 2012-501294 A (KUREHA CORPORATION) 19 January 2012 (2012-01-19), entire text | 1-5 |
| A | WO 2012/169516 A1 (KUREHA CORPORATION) 13 December 2012 (2012-12-13), entire text | 1-5 |

Form PCT/ISA/210 (second sheet) (January 2015)

**INTERNATIONAL SEARCH REPORT**
Information on patent family members

International application No.
PCT/JP2021/008690

| | | |
|---|---|---|
| JP 62-230771 A | 09 October 1987 | EP 236913 A2<br>DE 3608144 A<br>PT 84466 A<br>AU 6989587 A<br>DD 268852 A<br>DD 272405 A<br>DK 125187 A<br>HU 45975 A<br>ZA 8701756 A<br>KR 10-1987-0008854 A<br>ZA 871756 B<br>DK 125187 A0 |
| WO 2019/093522 A1 | 16 May 2019 | KR 10-2020-0013085 A<br>CN 111032631 A<br>CA 3071569 A<br>EA 202090456 A<br>AU 2018365928 B<br>IL 273066 D |
| JP 2014-520832 A | 25 August 2014 | US 2014/0155262 A1<br>entire text<br>US 2017/0081296 A1<br>WO 2013/007767 A1<br>EP 2731935 A1<br>AU 2012282501 A<br>CA 2840286 A<br>CN 103649057 A<br>CO 6890103 A<br>KR 10-2014-0022483 A<br>CR 20130673 A<br>IL 230031 A<br>MX 2014000366 A<br>EA 201400125 A<br>PE 8262014 A<br>MA 35344 B<br>CN 105152899 A<br>NZ 619937 A<br>UA 110843 C<br>ES 2570187 T<br>DK 2731935 T<br>PL 2731935 T<br>HU E027461 T<br>ZA 201401034 B<br>BR 112014000625 A<br>CL 2014000067 A<br>LT PA2019513 I<br>PE 20140826 A<br>UY 34203 A<br>AR 87194 A |

Form PCT/ISA/210 (patent family annex) (January 2015)

**INTERNATIONAL SEARCH REPORT**
Information on patent family members

International application No.
PCT/JP2021/008690

| | | |
|---|---|---|
| JP 54-027563 A | 01 March 1979 | US 4413003 A<br>entire text |
| JP 11-080127 A | 26 March 1999 | (Family: none) |
| JP 2012-501294 A | 19 January 2012 | US 2011/0124877 A1<br>entire text<br>WO 2010/023862 A1<br>EP 2315752 A1<br>AR 73130 A<br>AU 2009285455 A<br>CA 2726714 A<br>KR 10-2011-0036766 A<br>CN 102131786 A<br>BR PI0917461 A |
| WO 2012/169516 A1 | 13 December 2012 | US 2014/0179517 A1<br>entire text<br>US 2015/0299144 A1<br>EP 2757097 A1<br>CA 2838586 A<br>AU 2012268211 A<br>CN 103562187 A<br>KR 10-2014-0011406 A<br>EA 201391775 A<br>BR 112013031356 A<br>UA 110647 C |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013007767 A
- WO 2019093522 A
- WO 0364572 A


### Non-patent literature cited in the description


- *Org. Syn. Coll.,* 1990, vol. 7, 332
- *J. Med. Chem.,* 1995, vol. 38 (11), 1892-903
- *J. Heterocyc. Chem.,* 1981, vol. 18 (7), 1305-8
- *J. Am. Chem. Soc.,* 2001, vol. 123 (25), 5692-5973
- *CHEMICAL ABSTRACTS,* 887947-29-7